# EUROPEAN PATENT SPECIFICATION

(11) **EP 2 670 397 B1**
(45) Date of publication and mention of the grant of the patent: **13.05.2020**
(21) Application number: 12704975.7
(22) Date of filing: 31.01.2012
(51) Int. Cl.: A61K 31/198, A61K 31/351, A61K 31/403, A61K 31/4439, A61K 31/4985, A61K 31/64, A61K 9/20, A61K 9/28, A61K 31/155, A61K 9/24

(54) **PHARMACEUTICAL FORMULATIONS INCLUDING AN AMINE COMPOUND**
PHARMAZEUTISCHE FORMULIERUNGEN MIT EINER AMINVERBINDUNG
FORMULATIONS PHARMACEUTIQUES COMPRENANT UN COMPOSÉ AMINE

(30) Priority: 01.02.2011 US 201161438307 P
(43) Date of publication of application: 11.12.2013
(73) Proprietor: Bristol-Myers Squibb Company, Princeton, NJ 08543 (US); AstraZeneca UK Limited, London W1K 1LN (GB)
(72) Inventor: NARANG, Ajit, New Brunswick, NJ 08903 (US); RAO, Venkatramana, M., New Brunswick, NJ 08903 (US); DESAI, Divyakant, S., New Brunswick, NJ 08903 (US)
(74) Representative: Mewburn Ellis LLP
(86) International application number: PCT/US2012/023262
(87) International publication number: WO 2012/106303

(56) References cited:
- WO-A1-2005/117841
- WO-A1-2009/111200
- WO-A2-2009/121945
- GLASTRUP J: "Stabilisation of polyethylene and polypropylene glycol through inhibition of a beta-positioned hydroxyl group relative to an ether group. A study of modified triethylene and tripropylene glycols", POLYMER DEGRADATION AND STABILITY, BARKING, GB, vol. 81, no. 2, 1 January 2003 (2003-01-01), pages 273-278, XP004433133, ISSN: 0141-3910, DOI: 10.1016/S0141-3910(03)00097-1

## Description

### FIELD OF THE INVENTION

The present invention relates to coated tablets comprising amine compounds that can prevent or reduce formic acid and/or formyl species generation in the dosage form during the manufacturing process and/or during shelf-life storage. The formulations of the present invention can prevent or reduce formation of N-formyl impurities (and gelatin crosslinking) during the manufacturing process and/or during shelf-life storage.

### BACKGROUND OF THE INVENTION

Saxagliptin is a dipeptidyl peptidase IV (DPP-IV) inhibitor currently being used for the treatment of type 2 diabetes and is marketed under the trade name Onglyza®.
Saxagliptin tablets, 2.5 mg and 5 mg, have been developed using an active coating process. In this process, an inert tablet core is film-coated with three layers of pH-adjusted Opadry® II-based coating suspension. The first layer forms the seal-coat and contains Opadry® white; the second layer contains saxagliptin and Opadry® white; and the third, outer, layer contains Opadry® with optional color, to distinguish different strengths.

In addition, fixed dose combination (FDC) tablets of saxagliptin with metformin hydrochloride immediate release (Met IR) and extended release (Met XR) tablets have been developed using the same active coating process by replacing inert tablets cores with the Met IR or Met XR core tablets. The FDC tablets are intended to improve patient compliance by providing two drugs with different modes of action in a single tablet.

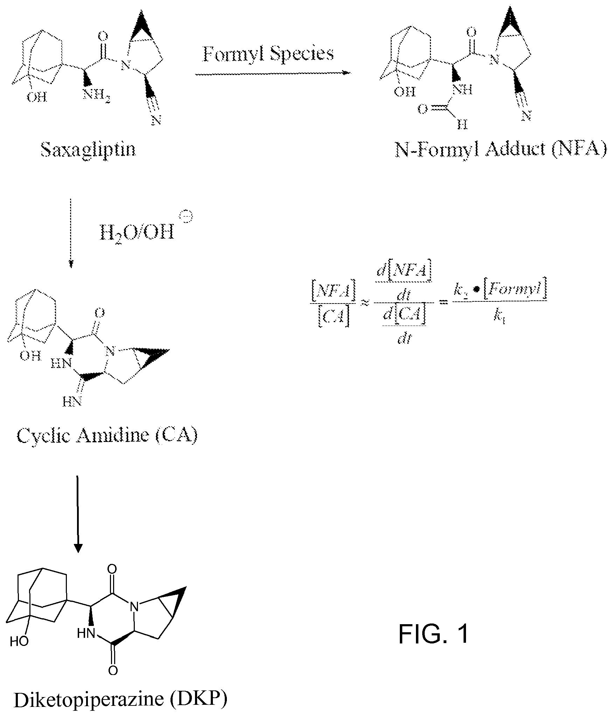

Figure 1 illustrates a pair of reaction paths for the degradation of saxagliptin. In tablet form, saxagliptin degrades via intra-molecular cyclization to form a cyclic amidine (CA) as described in WO 2005/117841. Formulating saxagliptin with Opadry® II white as the second coating reduces the formation of CA (and its reaction product DKP). However, the present inventors have determined that during the process of preparing the second coating, an N-formyl adduct (NFA) impurity is observed. The primary amine of saxagliptin is formylated by the reaction of saxagliptin with a formylating species such as formic acid, formic acid esters, formates, formic acid anhydrides and/or formaldehyde. Therefore, a need exists to prevent or reduce the degradation of saxagliptin during the manufacturing process as well as during shelf storage. WO 2005/117841 describes a saxagliptin tablet having the saxagliptin in the form of a hydrochloride salt disposed in an Opadry II-containing coating layer (i.e., which contains PEG and PVA).

### SUMMARY OF THE INVENTION

The present invention relates to coated tablets comprising:
(a) a tablet core wherein the tablet core comprises
   (i) optionally at least one antidiabetic agent or a pharmaceutically acceptable salt thereof selected from the group consisting of glyburide, metformin, rosiglitazone, pioglitazone, sitagliptin, vildagliptin, dapagliflozin, and dapagliflozin (S) propylene glycol hydrate;
(b) an active coating layer surrounding the tablet core wherein the coating layer comprises, in admixture,
   (i) a coating material comprising poly(ethylene glycol) and poly(vinyl alcohol);
   (ii) an amine compound that is glycine, histidine, arginine, ethanolamine, diethanolamine or triethanolamine, or a combination thereof; and
   (iii) saxagliptin or a pharmaceutically acceptable salt thereof.

The present invention thus generally relates to formulations comprising an amine compound that is glycine, histidine, arginine, ethanolamine, diethanolamine or triethanolamine, or a combination thereof that can prevent or reduce the formation of NFAs of saxagliptin or a pharmaceutically acceptable salt thereof. In particular, the formulations of the present invention can prevent or reduce formylation of saxagliptin during the manufacturing process and/or during shelf-life storage.

In one aspect, the present invention relates to coated tablet formulations comprising at least a tablet core and an active coating layer surrounding the tablet core wherein the coating layer comprises, in admixture, a coating material comprising poly(ethylene glycol) and poly(vinyl alcohol), one amine compound that is glycine, histidine, arginine, ethanolamine, diethanolamine or triethanolamine, or a combination thereof in combination with saxagliptin or a pharmaceutically acceptable salt thereof. The formulations of the present invention can prevent or reduce reaction of the active pharmaceutical ingredient with formylating species such as formaldehyde, formic acid, and/or derivatives thereof during the manufacturing process and/or during shelf storage, thereby preventing or reducing formyl impurities such as NFAs. The solid formulations of the present invention are tablet formulations. The coating formulations of the present invention have a propensity to form or support the formation of formaldehyde, formic acid, and/or derivatives thereof, for example during the manufacturing process.

In another aspect, the present invention relates to coated tablet formulations that contain a tablet core surrounded by an active coating layer. The tablet core may be inert, or may contain one or more antidiabetic agent(s). The tablet core is surrounded by an active coating layer that comprises an active pharmaceutical ingredient which is saxagliptin, in combination with a coating material and an amine compound such as glycine.

In another aspect, the present invention provides tablet formulations that contain a tablet core coated with three layers. The tablet core may be inert or may contain one or more antidiabetic agent(s). The first layer coats the tablet core and comprises a coating material and optionally an amine compound such as glycine. The second layer coats the first layer and comprises an active pharmaceutical ingredient which is saxagliptin, in combination with a coating material and an amine compound such as glycine. The third layer coats the second layer and comprises a coating material and optionally an amine compound such as glycine.

In another aspect, the present invention provides immediate release and extended release tablet formulations. The tablet core comprises an antidiabetic agent and three coatings. The first layer coats the tablet core and comprises a coating material and optionally an amine compound such as glycine. The second layer coats the first layer and comprises an active pharmaceutical ingredient which is saxagliptin, in combination with a coating material and an amine compound such as glycine. The third layer coats the second layer and comprises a coating material and optionally an amine compound such as glycine.

In another aspect, the present disclosure provides methods of preparing solid, semisolid, and liquid formulations that prevent or reduce N-formylation of an active pharmaceutical ingredient that is a primary amine or a secondary amine comprising adding an amine compound such as glycine to the formulation.

In another aspect, the present disclosure provides a method of preventing or reducing the formation of formic acid in solid, semisolid, or liquid formulations comprising adding an amine compound such as glycine to the formulation.

### BRIEF DESCRIPTION OF THE DRAWINGS

**Figure 1** illustrates reaction paths for certain degradation products of saxagliptin.
**Figure 2** illustrates possible degradation pathways of poly(ethylene glycol) to form formylating species.

### DETAILED DESCRIPTION OF THE DISCLOSURE

In certain aspects, the present invention relates to the use of an amine compoundthat is glycine, histidine, arginine, ethanolamine, diethanolamine or triethanolamine, or a combination thereof in coated tablet formulations to prevent or reduce N-formylation of amine-containing active pharmaceutical ingredients. Amine-containing active pharmaceutical ingredients, such as saxagliptin, can react with formic acid or its derivatives to form an NFA impurity. The amine compounds may react with formic acid or its derivatives to prevent or reduce N-formylation.

It has been surprisingly found that the presence of formic acid in a drug product impacts long-term drug product stability following short-term exposure of drug product to high temperature/humidity conditions or storage of the coating dispersion during drug product manufacture. As described in more detail below, many common formulation components, such as poly(ethylene glycol) (PEG) and poly(vinyl alcohol) (PVA) can include formic species in amounts sufficient to cause the undesirable formation of N-formyl adducts of primary or secondary amine-containing active pharmaceutical ingredients.

Reaction of a primary or secondary amine-containing active pharmaceutical ingredient with formic acid or a derivative thereof can form an N-formyl adduct. Factors that may contribute to N-formylation of active pharmaceutical ingredients include temperature, humidity, amount and type of formylating species present, and the microenvironment effects on solid-state reactivity. In the liquid state, a bimolecular reaction involving an amine-containing active pharmaceutical ingredient and a formylating species such as formic acid (or a derivative thereof such as formate) or formaldehyde is directly proportional to the concentrations of the reacting species. In the solid-state, temperature and moisture play a role by increasing the plasticity and molecular mobility of the reacting species. The origin of the formylating species may be attributed to degradation of the excipients during the process of manufacturing the tablet (or liquid formulation) or during storage.

Formyl impurities in drug products may in theory be controlled by preventing or reducing the presence of the formylating species in the formulations. However, there are significant challenges to controlling the level of formylating species generated during the manufacturing process and/or during shelf storage of feedstocks, coating materials, and finished dosage forms. For example, the type and relative proportion of formylating species are unknown. Among the species that may be present are formic acid, formates, acetic formic anhydride, and possibly other unknown species. These different species are expected to have different reactivities in a bimolecular reaction with an amine-containing active pharmaceutical ingredient.

Development of analytical methods for the quantification of formylating species is challenging. Methods for detecting formylating species typically require derivatization with an alcohol, such as ethanol, to form an ester followed by high performance liquid chromatography (HPLC) or gas chromatography (GC) separation and detection. These methods are generally non-specific with respect to type and relative proportion of formylating species present in the starting materials.

The formylating species may be present in the excipients even before the formulation process begins. For example, poly(vinyl alcohol), used in certain coatings described herein, is manufactured from poly(vinyl acetate) by hydrolysis. The PVA that is commercially available and typically used in film coating processes contains residual acetic acid. In addition, relatively high levels of formic acid can be present in the incoming PVA as an impurity. The presence of both acetic acid and formic acid in the coating formulation may facilitate formation of the highly reactive acetic formic anhydride.

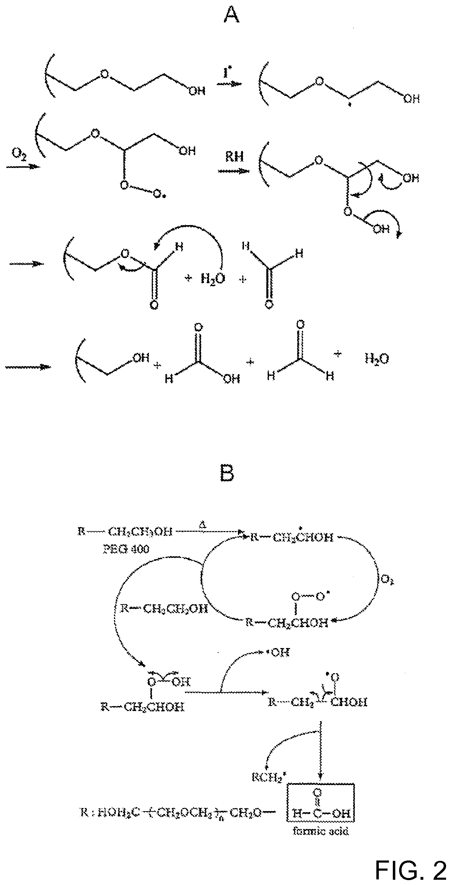

Formic acid is produced in poly(ethylene glycol) (PEG) upon exposure to high temperatures and humidity conditions and, without being bound by theory, is thought to be the result of an oxidative mechanism involving free radicals (Figure 2). In addition, levels of formic acid increase upon storage when PEG and PVA are combined, as compared to PEG alone.

In capsule dosage forms or capsule formulations, the formaldehyde generated from PEG reacts with gelatin to cross link the gelatin in addition to N-formylating the active ingredient. Crosslinked gelatin shells do not dissolve in aqueous medium easily, leading to slowdown or lack of capsule shell dissolution and therefore delayed release of the active ingredient. Cross linking of gelatin is encountered in hard gelatin capsule dosage forms including solid formulation ingredients that include higher (e.g., > 2000 Da) molecular weights of PEG. Cross linking is also encountered in liquid or semisolid filled soft gelatin formulations that comprise lower (e.g., < 2000 Da) molecular weights of PEGs that are liquid or semisolid at room temperature. The use of amine compounds in capsule shell formulations and/or drug product formulations can prevent or reduce capsule crosslinking and/or N-formylation of the active ingredient.

It has been found that the amount of formylating species can increase in tablet formulations during three different phases: shelf storage of coating materials as powders (e.g., containing PVA and/or PEG, such as certain Opadry® brand materials); shelf storage of the coated tablet (e.g., containing PVA and/or PEG in the solid-state); and manufacture of the tablet formulations (e.g., containing PVA and/or PEG in the solution state or the solid state), known as the suspension hold time. In addition, it has been found that N-formylation increases in tablets after an initial, short-term exposure of the tablet to high temperature/humidity conditions for a short period of time, for example as described below with respect to Example 1.

The initial, short term oxidation-promoting conditions that can cause initiation of a self-propagating chain reaction include the hold time storage of the Opadry® brand suspension. As described below with respect to Example 2, formyl species can increase relatively rapidly in Opadry® brand suspensions stored at room temperature. This initial increase in the formation of formyl species in the Opadry® suspension can have a negative effect on the drug product stability in finished packages. As described below with respect to Example 3, the storage of Opadry® brand suspension for up to 72 hours at room temperature during routine production operation could lead to higher NFA formation in the finished drug product.

Although BHT has been shown to effectively prevent formic acid growth in PEG in the solid-state, it may not be as effective in preventing formic acid generation in the solution state and in plasticized Opadry® films, which can have higher molecular mobility of reactive species as compared to the dry powder. Accordingly, use of an amine compound as described herein can offer significant advantages, as described below with respect to Examples 4-7, below.

The examples described herein show the tendency for formic acid generation during the storage of a PVA- and PEG-containing suspension (Opadry® II) and the long-term storage effects of initial, short term exposure to higher temperature and humidity conditions. The greater effectiveness of amine compounds such as glycine in preventing formation of NFAs of saxagliptin in Opadry® films is also shown. Effective inhibition formylation during the drug product manufacturing or an initial heat/moisture excursion can be critical to the long term, shelf-life storage stability of the drug product.

Accordingly, one aspect of the invention is a pharmaceutical dosage form including saxagliptin and an amine compound that is glycine, histidine, arginine, ethanolamine, diethanolamine or triethanolamine, or a combination thereof in substantial admixture. Saxagliptin and an amine compound that is glycine, histidine, arginine, ethanolamine, diethanolamine or triethanolamine, or a combination thereof are in intimate contact with one another, such that the chemical environment of each is substantially identical. Preferably, saxagliptin and an amine compound that is glycine, histidine, arginine, ethanolamine, diethanolamine or triethanolamine, or a combination thereof are homogenously mixed. Of course, there can in certain embodiments be some degree of inhomogeneity; for example, particles of the pharmaceutical ingredient and/or particles of the amine compound can be part of a substantially admixed material. The primary or secondary amine-containing active pharmaceutical ingredient and the amine compound can be disposed together, for example, in a tablet core, in a particulate or granulate formulation or in an active coating layer. The ratio of amine compound to primary or secondary amine-containing active pharmaceutical ingredient can be, for example, in the range of about 1:1 to about 100:1 on a molar basis. In certain embodiments, the ratio of amine compound to primary or secondary amine-containing active pharmaceutical ingredient is in the range of about 1:1 to about 20:1, in the range of about 2:1 to about 50:1, about 1:1 to about 10:1, about 2:1 to about 20:1, or even in the range of about 2:1 to about 10:1 on a molar basis.

According to the invention, saxagliptin and the amine compound that is glycine, histidine, arginine, ethanolamine, diethanolamine or triethanolamine, or a combination thereof are in substantial admixture with a poly(ethylene glycol) and a poly(vinyl alcohol). For example, in certain embodiments, the amine compound and the primary or secondary amine-containing active pharmaceutical ingredient are part of substantially admixed material that is at least about 10%, at least about 25%, or even at least about 50% poly(ethylene glycol), poly(vinyl alcohol), or a combination thereof (calculated on a wt/wt basis, excluding solvent). In one embodiment of the invention the active coating layer is at least 25% or at least about 50% total of poly(ethylene glycol) and poly(vinyl alcohol). In certain embodiments, the primary or secondary amine-containing active pharmaceutical ingredient and the amine compound are in substantial admixture with a poly(ethylene glycol), a poly(vinyl alcohol), or both, as is the case in the active coating layers based on Opadry® II material as described below.

A variety of amine compounds can be used in practicing the present invention. For example, in certain embodiments, organic amine compounds such as amino acids and amino alcohols can be used, and the amine compound can be, for example, a primary amine or a secondary amine. In certain embodiments, the amine compound is a primary amine. In certain embodiments, the amine compound is an amino acid. Examples of amino acids that can be used according to the invention are glycine, histidine and arginine. Amino alcohols suitable for use according to certain embodiments of the invention include ethanolamine, diethanolamine and triethanolamine. As described in more detail below, one preferred amine compound is glycine. Of course, other amine compounds (e.g., ammonium salts, EDTA) can be used in practicing the present invention. The amine compound can be provided, for example, in its free base form, substantially in an ammonium salt form, or as a combination of the two. As the person of skill in the art will appreciate, the amine compound can be provided as a combination of different amine compounds.

In certain embodiments, the amine compound is glycine. Surprisingly, the present inventors have determined that the use of glycine can provide greater protection from the formation of NFAs than other amino acids with more than one amine group and/or higher pKa values. Moreover, glycine can surprisingly provide effective protection from formation of NFAs even when it is provided substantially as an ammonium salt (e.g., when deposited from aqueous suspension having pH ∼ 2.0).

In certain embodiments of the invention, the amine compound is provided at a concentration in the range of 0.01 wt% to 10 wt% of the coating layer. In preferred embodiments of the invention, the amine compound is provided at a concentration in the range of 0.5 wt% to 5 wt% of the coating layer. For example, the amine compound can be provided at a concentration in the range of about 0.1 wt% to about 5 wt% of the material in which it is disposed.

In certain embodiments, the primary or secondary amine-containing active pharmaceutical ingredient is saxagliptin, sitagliptin, vildagliptin, linagliptin, dutogliptin, or alogliptin. For example, in one embodiment, the active pharmaceutical ingredient is saxagliptin. The active pharmaceutical ingredient can be provided as its free base, as a pharmaceutically acceptable salt, or as a combination thereof. For example, the active pharmaceutical ingredient can be substantially provided as an ammonium salt or as a hydrochloride salt. In one embodiment of the invention saxagliptin is substantially provided as an ammonium salt.

In certain embodiments of the present invention the coating layer also comprises a water-soluble antioxidant(s) in combination with the amine compound and the primary or secondary amine-containing active pharmaceutical ingredient. The water soluble antioxidant can be, for example, ascorbic acid, propyl gallate, sodium sulfite, sodium metabisulfite, sodium bisulfite, thioglycerol, thioglycollic acid, or a combination thereof. In certain embodiments of the invention, the water soluble antioxidant is ascorbic acid or propyl gallate. The use of water soluble antioxidants is detailed in U.S. Provisional Patent Application serial no. 61/379,970.

Certain embodiments of the invention as described above are described in further detail below with respect to a variety of dosage forms.

In another aspect, the present disclosure provides a method of preventing or reducing N-formylation of active pharmaceutical ingredients in a substantially admixed solid, semisolid, or liquid formulation material comprising adding an amine compound to the formulation material. The solid, semisolid, or liquid formulation materials can comprise a poly(ethylene glycol), a poly(vinyl alcohol), or a combination thereof. For example, in certain embodiments, the formulations include poly(ethylene glycol), optionally in combination with poly(vinyl alcohol), as is the case in the Opadry® II material. The active pharmaceutical ingredient is a primary or secondary amine. Preferred active pharmaceutical ingredients are selected from saxagliptin, sitagliptin, vildagliptin, linagliptin, dutogliptin, and alogliptin. The most preferred active pharmaceutical ingredient is saxagliptin. Preferred amine compounds include those described above. The most preferred amine compound is glycine. The teachings of the present disclosure as described with respect to dosage forms can be applied to the methods of the present disclosure by the person of skill in the art.

### Coated Tablet Embodiments

The present invention relates to tablet formulations that comprise a tablet core. An active coating layer surrounds the tablet core, and comprises, in admixture, a coating material comprising poly(ethylene glycol) and poly(vinyl alcohol), an amine compound that is glycine, histidine, arginine, ethanolamine, diethanolamine or triethanolamine, or a combination thereof, and saxagliptin or a pharmaceutically acceptable salt thereof. The active coating layer can be provided as described above, e.g., with the primary or secondary amine-containing active pharmaceutical ingredient and amine compound as part of substantially admixed material that is at least about 10%, at least about 25%, or even at least about 50% poly(ethylene glycol), poly(vinyl alcohol), or a combination thereof. An example formulation is provided below in Table 15.

The tablet core can in certain embodiments include a drug different from the primary or secondary amine-containing active pharmaceutical ingredient. For example, in one embodiment, the tablet core optionally comprises at least one antidiabetic or a pharmaceutically acceptable salt thereof, where the antidiabetic is other than the primary or secondary amine-containing active pharmaceutical ingredient (e.g., other than saxagliptin). The antidiabetic can be, for example, an alpha glucosidase inhibitor, insulin, a meglitinide, a sulfonylurea, a biguanide, a biguanide/glyburide combination, a thiazolidinedione, a PPAR-alpha agonist, a PPAR-gamma agonist, a PPAR alpha/gamma dual agonist, a glycogen phosphorylase inhibitor, an inhibitor of fatty acid binding protein (aP2), a GPR-119 modulator, a GPR 40 modulator, a glucokinase inhibitor, a glucagon-like peptide-1 (GLP-1) or another agonist of the GLP-1 receptor, a SGLT2 inhibitor, dipeptidyl peptidase IV (DPP4) inhibitor other than saxagliptin, or combinations thereof. For example, the antidiabetic can be acarbose, miglitol, insulin, repaglinide, nateglinide, KAD1229, acetohexamide, chlorpropamide, glibenclamide (glyburide), gliclazide, glimepiride, glipizide, glyclopyramide, tolazamide, tolbutamide, buformin, metformin, phenformin, Glucovance®, rosiglitazone, pioglitazone, troglitazone, MCC-555, faraglitazar, englitazone, darglitazone, CP-86325, isaglitazone, reglitazar, JTT-501, rivoglitazone, R-119702, liraglutide, (Z)-1,4-bis-4-[(3,5-dioxo-1,2,4-oxadiazolidin-2-yl-methyl)]phenoxybut-2-ene, YM-440, , muraglitazar, peliglitazar, tesaglitazar AR-HO39242, GW-501516, KRP297, sitagliptin, vildagliptin, linagliptin, dutogliptin, and alogliptin, NVP-DPP728A (1-[[[2-[(5-cyanopyridin-2-yl)amino]ethyl]amino]acetyl]-2-cyano-(S)-pyrrolidine), TSL-225 (tryptophyl-1,2,3,4-tetrahydroisoquinoline-3-carboxylic acid, canagliflozin, dapagliflozin, dapagliflozin (S) propylene glycol hydrate, remogliflozin, sergliflozin. In one embodiment, metformin is provided in the tablet core. In another embodiment, dapagliflozin (e.g., as dapagliflozin (S) propylene glycol hydrate) is provided in the tablet core. In other embodiments, the tablet core is inert (e.g., contains substantially no drug).

In another aspect, the present invention provides tablet formulations that comprise a tablet core that optionally comprises at least one antidiabetic or a pharmaceutically acceptable salt thereof selected from the group consisting of glyburide, metformin, rosiglitazone, pioglitazone, sitagliptin, vildagliptin, dapagliflozin, and dapagliflozin (S) propylene glycol hydrate. A first layer coats the tablet core and comprises a coating material and optionally an amine compound. The active coating layer described herein is provided as a second layer coating the first layer and comprises an active pharmaceutical ingredient or a pharmaceutically acceptable salt thereof, a coating material, and an amine compound that is glycine, histidine, arginine, ethanolamine, diethanolamine or triethanolamine, or a combination thereof, where the active pharmaceutical ingredient is saxagliptin. A third layer coats the second layer and comprises a coating material and optionally an amine compound.

In another aspect, the present invention provides tablet formulations that comprise a tablet core that optionally comprises at least one antidiabetic or a pharmaceutically acceptable salt thereof, where the antidiabetic is other than saxagliptin. A first layer coats the tablet core and comprises a coating material and optionally an amine compound. The active coating layer described herein is provided as a second layer coating the first layer and comprises an active pharmaceutical ingredient, a coating material, and an amine compound that is glycine, histidine, arginine, ethanolamine, diethanolamine or triethanolamine, or a combination thereof, where the active pharmaceutical ingredient is saxagliptin. A third layer coats the second layer and comprises a coating material and optionally an amine compound.

In another aspect, the present invention provides tablet formulations that comprise a tablet core that optionally comprises at least one antidiabetic agent or a pharmaceutically acceptable salt thereof selected from the group consisting of glyburide, metformin, rosiglitazone, pioglitazone, sitagliptin, vildagliptin, dapagliflozin, and dapagliflozin (S) propylene glycol hydrate. A first layer coats the tablet core and comprises a coating material and optionally an amine compound. The active coating layer described herein is provided as a second layer coating the first layer and comprises an active pharmaceutical ingredient, a coating material, and an amine compound that is glycine, histidine, arginine, ethanolamine, diethanolamine or triethanolamine, or a combination thereof, where the active pharmaceutical ingredient is saxagliptin. A third layer coats the second layer and comprises a coating material and optionally an amine compound.

The coating material of one or more of the layers (e.g., the active coating layer, the first layer, the second layer and/or the third layer) can in certain embodiments include a poly(ethylene glycol), a poly(vinyl alcohol), or a combination thereof. For example, in certain embodiments, the coating material includes a poly(ethylene glycol) in the range of about 10 wt% to about 40 wt%, and a poly(vinyl alcohol) in the range of about 25 wt% to about 60 wt%. The coating material can optionally include other materials, such as glidants (e.g., talc), pigments (e.g., titanium dioxide), colarants and plasticizers. Certain suitable coating materials are available under the trade name Opadry®.

In another aspect, the present invention provides tablet formulations that comprise a tablet core that optionally comprises at least one antidiabetic or a pharmaceutically acceptable salt thereof, selected from the group consisting of glyburide, metformin, rosiglitazone, pioglitazone, sitagliptin, vildagliptin, dapagliflozin, and dapagliflozin (S) propylene glycol hydrate. A first layer coats the tablet core and comprises Opadry® II and optionally an amine compound. The active coating layer described herein is provided as a second layer coating the first layer and comprises an active pharmaceutical ingredient, Opadry® II, and an amine compound that is glycine, histidine, arginine, ethanolamine, diethanolamine or triethanolamine, or a combination thereof, where the active pharmaceutical ingredient is saxagliptin. A third layer coats the second layer and comprises Opadry® II and optionally an amine compound.

In another aspect, the present invention provides tablet formulations that comprise a tablet core that optionally comprises at least one antidiabetic or a pharmaceutically acceptable salt thereof, selected from the group consisting of glyburide, metformin, rosiglitazone, pioglitazone, sitagliptin, vildagliptin, dapagliflozin, and dapagliflozin (S) propylene glycol hydrate. A first layer coats the tablet core and comprises a coating material and optionally an amine compound. The active coating layer described herein is provided as a second layer coating the first layer and comprises an active pharmaceutical ingredient, a coating material, and an amine compound, where the active pharmaceutical ingredient is saxagliptin. A third layer coats the second layer and comprises a coating material and optionally an amine compound. The amine compound of each layer is a primary amine or a secondary amine, for example, an amino acid such as glycine.

In another aspect, the present invention provides tablet formulations that comprise a tablet core that optionally comprises at least one antidiabetic or a pharmaceutically acceptable salt thereof, selected from the group consisting of glyburide, metformin, rosiglitazone, pioglitazone, sitagliptin, vildagliptin, dapagliflozin, and dapagliflozin (S) propylene glycol hydrate. A first layer coats the tablet core and comprises Opadry® II and optionally an amine compound. The active coating layer described herein is provided as a second layer coating the first layer and comprises an active pharmaceutical ingredient, Opadry® II, and an amine compound, where the active pharmaceutical ingredient is saxagliptin. A third layer coats the second layer and comprises Opadry® II and optionally an amine compound. The amine compound of each layer is a primary amine or a secondary amine, for example, an amino acid such as glycine.

In another aspect, the present invention provides tablet formulations that comprise a tablet core that optionally comprises at least one antidiabetic or a pharmaceutically acceptable salt thereof, selected from the group consisting of glyburide, metformin, rosiglitazone, pioglitazone, sitagliptin, vildagliptin, dapagliflozin, and dapagliflozin (S) propylene glycol hydrate. A first layer coats the tablet core and comprises a coating material and optionally an amine compound. The active coating layer described herein is provided as a second layer coating the first layer and comprises an active pharmaceutical ingredient, a coating material, and an amine compound, where the active pharmaceutical ingredient is saxagliptin. A third layer coats the second layer and comprises a coating material and optionally an amine compound. The amine compound in each layer is glycine.

In another aspect, the present invention provides tablet formulations that comprise a tablet core that optionally comprises at least one antidiabetic or a pharmaceutically acceptable salt thereof, selected from the group consisting of glyburide, metformin, rosiglitazone, pioglitazone, sitagliptin, vildagliptin, dapagliflozin, and dapagliflozin (S) propylene glycol hydrate. A first layer coats the tablet core and comprises Opadry® II and optionally an amine compound. The active coating layer described herein is provided as a second layer coating the first layer and comprises an active pharmaceutical ingredient, Opadry® II, and an amine compound, where the active pharmaceutical ingredient is saxagliptin. A third layer coats the second layer and comprises Opadry® II and optionally an amine compound. The amine compound in each layer is glycine.

In another aspect, the present invention provides tablet formulations that comprise a tablet core that comprises one or more fillers, a disintegrant, and a lubricant. A first layer coats the tablet core and comprises Opadry® II and optionally an amine compound. The active coating layer described herein is provided as a second layer coating the first layer and comprises an active pharmaceutical ingredient, Opadry® II, and an amine compound, where the active pharmaceutical ingredient is saxagliptin. A third layer coats the second layer and comprises Opadry® II and optionally an amine compound. The amine compound in each layer is glycine.

In another aspect, the present invention provides tablet formulations that comprise a tablet core that comprises two fillers, a disintegrant, and a lubricant. A first layer coats the tablet core and comprises Opadry® II. The active coating layer described herein is provided as a second layer coating the first layer and comprises an active pharmaceutical ingredient, Opadry® II, and an amine compound, where the active pharmaceutical ingredient is saxagliptin and the amine compound is glycine. A third layer coats the second layer and comprises Opadry® II.

In another aspect, the present invention provides tablet formulations that comprise a tablet core that comprises two fillers, a disintegrant, and a lubricant. A first layer coats the tablet core and comprises Opadry® II. The active coating layer described herein is provided as a second layer coating the first layer and comprises an active pharmaceutical ingredient, Opadry® II, and an amine compound, where the active pharmaceutical ingredient is saxagliptin and the amine compound is glycine. A third layer coats the second layer and comprises Opadry® II.

In another aspect, the present invention provides tablet formulations that comprise a tablet core that comprises two fillers, a disintegrant, and a lubricant. A first layer coats the tablet core and comprises Opadry® II. The active coating layer described herein is provided as a second layer coating the first layer and comprises an active pharmaceutical ingredient, Opadry® II, and an amine compound, where the active pharmaceutical ingredient is saxagliptin and the amine compound is glycine. A third layer coats the second layer and comprises Opadry® II.

In another aspect, the present invention provides tablet formulations that comprise a tablet core that comprises lactose monohydrate, microcrystalline cellulose, croscarmellose sodium, and magnesium stearate. A first layer coats the tablet core and comprises Opadry® II white. The active coating layer described herein is provided as a second layer coating the first layer and comprises saxagliptin, Opadry® II white, and glycine. A third layer coats the second layer and comprises Opadry® II color.

In another aspect, the present invention provides tablet formulations that comprise a tablet core that comprises lactose monohydrate, microcrystalline cellulose, croscarmellose sodium, and magnesium stearate. A first layer coats the tablet core and comprises Opadry® II white. The active coating layer described herein is provided as a second layer coating the first layer and comprises saxagliptin, Opadry® II white, and glycine. A third layer coats the second layer and comprises Opadry® II color.

In another aspect, the present invention provides tablet formulations that comprise a tablet core that comprises lactose monohydrate, microcrystalline cellulose, croscarmellose sodium, and magnesium stearate. A first layer coats the tablet core and comprises Opadry® II white. The active coating layer described herein is provided as a second layer coating the first layer and comprises saxagliptin, Opadry® II white, and glycine. A third layer coats the second layer and comprises Opadry® II color.

### Immediate Release Embodiments

The coated tablet embodiments described above can be modified to provide immediate release formulations. Accordingly, in another aspect, the present invention provides immediate release tablet formulations that comprise a tablet core that comprises a binder, a wetting agent, a lubricant, and optionally at least one antidiabetic or a pharmaceutically acceptable salt thereof, where the antidiabetic is other than saxagliptin. A first layer coats the tablet core and comprises a coating material and optionally an amine compound. The active coating layer described herein is provided as a second layer coating the first layer and comprises an active pharmaceutical ingredient, a coating material, and an amine compound that is glycine, histidine, arginine, ethanolamine, diethanolamine or triethanolamine, or a combination thereof, where the active pharmaceutical ingredient is saxagliptin. A third layer coats the second layer and comprises a coating material and optionally an amine compound. An example formulation is provided below in Table 16.

In another aspect, the present invention provides immediate release tablet formulations that comprise a tablet core that comprises a binder, a wetting agent, a lubricant, and at least one antidiabetic or a pharmaceutically acceptable salt thereof, where the antidiabetic is an alpha glucosidase inhibitor, insulin, a meglitinide, a sulfonylurea, a biguanide, a biguanide/glyburide combination, a thiazolidinedione, a PPAR-alpha agonist, a PPAR-gamma agonist, a PPAR alpha/gamma dual agonist, a glycogen phosphorylase inhibitor, an inhibitor of fatty acid binding protein (aP2), a GPR-119 modulator, a GPR 40 modulator, a glucokinase inhibitor, a glucagon-like peptide-1 (GLP-1) or another agonist of the GLP-1 receptor, a SGLT2 inhibitor, dipeptidyl peptidase IV (DPP4) inhibitor other than saxagliptin, or combinations thereof. A first layer coats the tablet core and comprises a coating material and optionally an amine compound. The active coating layer described herein is provided as a second layer coating the first layer and comprises an active pharmaceutical ingredient, a coating material, and an amine compound that is glycine, histidine, arginine, ethanolamine, diethanolamine or triethanolamine, or a combination thereof, where the active pharmaceutical ingredient is saxagliptin. A third layer coats the second layer and comprises a coating material and optionally an amine compound.

In another aspect, the present invention provides immediate release tablet formulations that comprise a tablet core that comprises a binder, a wetting agent, a lubricant, and at least one antidiabetic or a pharmaceutically acceptable salt thereof, where the antidiabetic is acarbose, miglitol, insulin, repaglinide, nateglinide, KAD1229, acetohexamide, chlorpropamide, glibenclamide (glyburide), gliclazide, glimepiride, glipizide, glyclopyramide, tolazamide, tolbutamide, buformin, metformin, phenformin, Glucovance®, rosiglitazone, pioglitazone, troglitazone, MCC-555, faraglitazar, englitazone, darglitazone, CP-86325, isaglitazone, reglitazar, JTT-501, rivoglitazone, R-119702, liraglutide, (Z)-1,4-bis-4-[(3,5-dioxo-1,2,4-oxadiazolidin-2-yl-methyl)]phenoxybut-2-ene, YM-440, , muraglitazar, peliglitazar, tesaglitazar AR-HO39242, GW-501516, KRP297, sitagliptin, vildagliptin, linagliptin, dutogliptin, and alogliptin, NVP-DPP728A (1-[[[2-[(5-cyanopyridin-2-yl)amino]ethyl]amino]acetyl]-2-cyano-(S)-pyrrolidine), TSL-225 (tryptophyl-1,2,3,4-tetrahydroisoquinoline-3-carboxylic acid, canagliflozin, dapagliflozin, dapagliflozin (S) propylene glycol hydrate, remogliflozin, sergliflozin or combinations thereof. A first layer coats the tablet core and comprises a coating material and optionally an amine compound. The active coating layer described herein is provided as a second layer coating the first layer coats the first layer and comprises an active pharmaceutical ingredient, a coating material, and an amine compound that is glycine, histidine, arginine, ethanolamine, diethanolamine or triethanolamine, or a combination thereof, where the active pharmaceutical ingredient is saxagliptin. A third layer coats the second layer and comprises a coating material and optionally an amine compound.

In another aspect, the present invention provides immediate release tablet formulations that comprise a tablet core that comprises a binder, a wetting agent, a lubricant, and at least one antidiabetic or a pharmaceutically acceptable salt thereof, where the antidiabetic is glibenclamide (glyburide), metformin, Glucovance®, rosiglitazone, pioglitazone, sitagliptin, vildagliptin, dapagliflozin, dapagliflozin (S) propylene glycol hydrate, or combinations thereof. A first layer coats the tablet core and comprises a coating material and optionally an amine compound. The active coating layer described herein is provided as a second layer coating the first layer and comprises an active pharmaceutical ingredient, a coating material, and an amine compound that is glycine, histidine, arginine, ethanolamine, diethanolamine or triethanolamine, or a combination thereof, where the active pharmaceutical ingredient is saxagliptin. A third layer coats the second layer and comprises a coating material and optionally an amine compound.

In another aspect, the present invention provides immediate release tablet formulations that comprise a tablet core that comprises a binder, a wetting agent, a lubricant, and at least one antidiabetic or a pharmaceutically acceptable salt thereof, where the antidiabetic is glyburide, metformin, Glucovance®, rosiglitazone, pioglitazone, sitagliptin, vildagliptin, dapagliflozin, dapagliflozin (S) propylene glycol hydrate, or combinations thereof. A first layer coats the tablet core and comprises a coating material and optionally an amine compound. The active coating layer described herein is provided as a second layer coating the first layer and comprises an active pharmaceutical ingredient, a coating material, and an amine compound that is glycine, histidine, arginine, ethanolamine, diethanolamine or triethanolamine, or a combination thereof, where the active pharmaceutical ingredient is saxagliptin. A third layer coats the second layer and comprises a coating material and optionally an amine compound.

In another aspect, the present invention provides immediate release tablet formulations that comprise a tablet core that comprises a binder, a wetting agent, a lubricant, and at least one antidiabetic or a pharmaceutically acceptable salt thereof, where the antidiabetic is glyburide, metformin, Glucovance®, rosiglitazone, pioglitazone, sitagliptin, vildagliptin, dapagliflozin, dapagliflozin (S) propylene glycol hydrate, or combinations thereof. A first layer coats the tablet core and comprises Opadry® II and optionally an amine compound. The active coating layer described herein is provided as a second layer coating the first layer and comprises an active pharmaceutical ingredient, Opadry® II, and an amine compound that is glycine, histidine, arginine, ethanolamine, diethanolamine or triethanolamine, or a combination thereof, where the active pharmaceutical ingredient is saxagliptin. A third layer coats the second layer and comprises Opadry® II and optionally an amine compound.

In another aspect, the present invention provides immediate release tablet formulations that comprise a tablet core that comprises a binder, a wetting agent, a lubricant, and at least one antidiabetic or a pharmaceutically acceptable salt thereof, where the antidiabetic is metformin, dapagliflozin, dapagliflozin (S) propylene glycol hydrate, or combinations thereof. A first layer coats the tablet core and comprises a coating material and optionally an amine compound. The active coating layer described herein is provided as a second layer coating the first layer and comprises an active pharmaceutical ingredient, a coating material, and an amine compound that is glycine, histidine, arginine, ethanolamine, diethanolamine or triethanolamine, or a combination thereof, where the active pharmaceutical ingredient is saxagliptin. A third layer coats the second layer and comprises a coating material and optionally an amine compound.

In another aspect, the present invention provides immediate release tablet formulations that comprise a tablet core that comprises a binder, a wetting agent, a lubricant, and at least one antidiabetic or a pharmaceutically acceptable salt thereof, where the antidiabetic is metformin, dapagliflozin, dapagliflozin (S) propylene glycol hydrate, or combinations thereof. A first layer coats the tablet core and comprises Opadry® II and optionally an amine compound. The active coating layer described herein is provided as a second layer coating the first layer and comprises an active pharmaceutical ingredient, Opadry® II, and an amine compound that is glycine, histidine, arginine, ethanolamine, diethanolamine or triethanolamine, or a combination thereof, where the active pharmaceutical ingredient is saxagliptin. A third layer coats the second layer and comprises Opadry® II and optionally an amine compound.

In another aspect, the present invention provides immediate release tablet formulations that comprise a tablet core that comprises a binder, a wetting agent, a lubricant, and at least one antidiabetic or a pharmaceutically acceptable salt thereof, where the antidiabetic is metformin, dapagliflozin, dapagliflozin (S) propylene glycol hydrate, or combinations thereof. A first layer coats the tablet core and comprises a coating material and optionally an amine compound. The active coating layer described herein is provided as a second layer coating the first layer and comprises an active pharmaceutical ingredient, a coating material, and an amine compound, where the active pharmaceutical ingredient is saxagliptin. A third layer coats the second layer and comprises a coating material and optionally a water soluble antioxidant. The amine compound in each layer can be as described above, for example, glycine, histidine, arginine, ethanolamine, diethanolamine or triethanolamine, or a combination thereof.

In another aspect, the present invention provides immediate release tablet formulations that comprise a tablet core that comprises a binder, a wetting agent, a lubricant, and at least one antidiabetic or a pharmaceutically acceptable salt thereof, where the antidiabetic is metformin, dapagliflozin, dapagliflozin (S) propylene glycol hydrate, or combinations thereof. A first layer coats the tablet core and comprises Opadry® II and optionally an amine compound. The active coating layer described herein is provided as a second layer coating the first layer and comprises an active pharmaceutical ingredient, Opadry® II, and an amine compound, where the active pharmaceutical ingredient is saxagliptin. A third layer coats the second layer and comprises Opadry® II and optionally an amine compound. The amine compound in each layer can be as described above, for example, glycine, histidine, arginine, ethanolamine, diethanolamine or triethanolamine, or a combination thereof.

In another aspect, the present invention provides immediate release tablet formulations that comprise a tablet core that comprises a binder, a wetting agent, a lubricant, and at least one antidiabetic or a pharmaceutically acceptable salt thereof, where the antidiabetic is metformin, dapagliflozin, dapagliflozin (S) propylene glycol hydrate, or combinations thereof. A first layer coats the tablet core and comprises Opadry® II and optionally an amine compound. The active coating layer described herein is provided as a second layer coating the first layer and comprises an active pharmaceutical ingredient, Opadry® II, and an amine compound, where the active pharmaceutical ingredient is saxagliptin. A third layer coats the second layer and comprises Opadry® II and optionally an amine compound. The amine compound in each layer is glycine.

In another aspect, the present invention provides immediate release tablet formulations that comprise a tablet core that comprises a binder, a wetting agent, a lubricant, and dapagliflozin or dapagliflozin (S) propylene glycol hydrate. A first layer coats the tablet core and comprises Opadry® II. The active coating layer described herein is provided as a second layer coating the first layer and comprises saxagliptin, Opadry® II, and glycine. A third layer coats the second layer and comprises Opadry® II.

In another aspect, the present invention provides immediate release tablet formulations that comprise a tablet core that comprises povidone, purified water, magnesium stearate, and dapagliflozin or dapagliflozin (S) propylene glycol hydrate. A first layer coats the tablet core and comprises Opadry® II white. The active coating layer described herein is provided as a second layer coating the first layer and comprises saxagliptin, Opadry® II white, and glycine. A third layer coats the second layer and comprises Opadry® II color.

In another aspect, the present invention provides immediate release tablet formulations that comprise a tablet core that comprises a binder, a wetting agent, a lubricant, and metformin hydrochloride. A first layer coats the tablet core and comprises Opadry® II white. The active coating layer described herein is provided as a second layer coating the first layer and comprises saxagliptin, Opadry® II white, and glycine. A third layer coats the second layer and comprises Opadry®II color.

In another aspect, the present invention provides immediate release tablet formulations that comprise a tablet core that comprises povidone, purified water, magnesium stearate, and metformin hydrochloride. A first layer coats the tablet core and comprises Opadry® II white. The active coating layer described herein is provided as a second layer coating the first layer and comprises saxagliptin, Opadry® II white, and glycine. A third layer coats the second layer and comprises Opadry® II color.

### Extended Release Embodiments

The coated tablet embodiments described above can be modified to provide extended release formulations. Accordingly, in another aspect, the present invention provides extended release tablet formulations that comprise a tablet core that comprises a disintegrant, a release modifier, a filler, a wetting agent, a lubricant, and optionally at least one antidiabetic or a pharmaceutically acceptable salt thereof, where the antidiabetic is other than saxagliptin. A first layer coats the tablet core and comprises a coating material and optionally an amine compound. The active coating layer described herein is provided as a second layer coating the first layer and comprises an active pharmaceutical ingredient, a coating material, and an amine compound that is glycine, histidine, arginine, ethanolamine, diethanolamine or triethanolamine, or a combination thereof, where the active pharmaceutical ingredient is saxagliptin. A third layer coats the second layer and comprises a coating material and optionally an amine compound. An example formulation is provided below in Table 17.

In another aspect, the present invention provides extended release tablet formulations that comprise a tablet core that comprises a disintegrant, a release modifier, a filler, a wetting agent, a lubricant, and at least one antidiabetic or a pharmaceutically acceptable salt thereof, where the antidiabetic is an alpha glucosidase inhibitor, insulin, a meglitinide, a sulfonylurea, a biguanide, a biguanide/glyburide combination, a thiazolidinedione, a PPAR-alpha agonist, a PPAR-gamma agonist, a PPAR alpha/gamma dual agonist, a glycogen phosphorylase inhibitor, an inhibitor of fatty acid binding protein (aP2), a GPR-119 modulators, a GPR 40 modulator, a glucokinase inhibitor, a glucagon-like peptide-1 (GLP-1) or another agonist of the GLP-1 receptor, a SGLT2 inhibitor, dipeptidyl peptidase IV (DPP4) inhibitor other than saxagliptin, or combinations thereof. A first layer coats the tablet core and comprises a coating material and optionally an amine compound. The active coating layer described herein is provided as a second layer coating the first layer and comprises an active pharmaceutical ingredient, a coating material, and an amine compound that is glycine, histidine, arginine, ethanolamine, diethanolamine or triethanolamine, or a combination thereof, where the active pharmaceutical ingredient is saxagliptin. A third layer coats the second layer and comprises a coating material and optionally an amine compound.

In another aspect, the present invention provides extended release tablet formulations that comprise a tablet core that comprises a disintegrant, a release modifier, a filler, a wetting agent, a lubricant, and at least one antidiabetic or a pharmaceutically acceptable salt thereof, where the antidiabetic is acarbose, miglitol, insulin, repaglinide, nateglinide, KAD1229, acetohexamide, chlorpropamide, glibenclamide (glyburide), gliclazide, glimepiride, glipizide, glyclopyramide, tolazamide, tolbutamide, buformin, metformin, phenformin, Glucovance®, rosiglitazone, pioglitazone, troglitazone, MCC-555, faraglitazar, englitazone, darglitazone, CP-86325, isaglitazone, reglitazar, JTT-501, rivoglitazone, R-119702, liraglutide, (Z)-1,4-bis-4-[(3,5-dioxo-1,2,4-oxadiazolidin-2-yl-methyl)]phenoxybut-2-ene, YM-440, , muraglitazar, peliglitazar, tesaglitazar AR-HO39242, GW-501516, KRP297, sitagliptin, vildagliptin, linagliptin, dutogliptin, and alogliptin, NVP-DPP728A (1-[[[2-[(5-cyanopyridin-2-yl)amino]ethyl]amino]acetyl]-2-cyano-(S)-pyrrolidine), TSL-225 (tryptophyl-1,2,3,4-tetrahydroisoquinoline-3-carboxylic acid, canagliflozin, dapagliflozin, dapagliflozin (S) propylene glycol hydrate, remogliflozin, sergliflozin or combinations thereof. A first layer coats the tablet core and comprises a coating material and optionally an amine compound. The active coating layer described herein is provided as a second layer coating the first layer and comprises an active pharmaceutical ingredient, a coating material, and an amine compound that is glycine, histidine, arginine, ethanolamine, diethanolamine or triethanolamine, or a combination thereof, where the active pharmaceutical ingredient is saxagliptin. A third layer coats the second layer and comprises a coating material and optionally an amine compound.

In another aspect, the present invention provides extended release tablet formulations that comprise a tablet core that comprises a disintegrant, a release modifier, a filler, a wetting agent, a lubricant, and at least one antidiabetic or a pharmaceutically acceptable salt thereof, where the antidiabetic is glibenclamide (glyburide), metformin, Glucovance®, rosiglitazone, pioglitazone, sitagliptin, vildagliptin, dapagliflozin, dapagliflozin (S) propylene glycol hydrate, or combinations thereof. A first layer coats the tablet core and comprises a coating material and optionally an amine compound. The active coating layer described herein is provided as a second layer coating the first layer coats the first layer and comprises an active pharmaceutical ingredient, a coating material, and an amine compound that is glycine, histidine, arginine, ethanolamine, diethanolamine or triethanolamine, or a combination thereof, where the active pharmaceutical ingredient is saxagliptin. A third layer coats the second layer and comprises a coating material and optionally an amine compound.

In another aspect, the present invention provides extended release tablet formulations that comprise a tablet core that comprises a disintegrant, a release modifier, a filler, a wetting agent, a lubricant, and at least one antidiabetic or a pharmaceutically acceptable salt thereof, where the antidiabetic is glyburide, metformin, Glucovance®, rosiglitazone, pioglitazone, sitagliptin, vildagliptin, dapagliflozin, dapagliflozin (S) propylene glycol hydrate, or combinations thereof. A first layer coats the tablet core and comprises a coating material and optionally an amine compound. The active coating layer described herein is provided as a second layer coating the first layer and comprises an active pharmaceutical ingredient, a coating material, and an amine compound that is glycine, histidine, arginine, ethanolamine, diethanolamine or triethanolamine, or a combination thereof, where the active pharmaceutical ingredient is saxagliptin. A third layer coats the second layer and comprises a coating material and optionally an amine compound.

In another aspect, the present invention provides extended release tablet formulations that comprise a tablet core that comprises a disintegrant, a release modifier, a filler, a wetting agent, a lubricant, and at least one antidiabetic or a pharmaceutically acceptable salt thereof, where the antidiabetic is glyburide, metformin, Glucovance®, rosiglitazone, pioglitazone, sitagliptin, vildagliptin, dapagliflozin, dapagliflozin (S) propylene glycol hydrate, or combinations thereof. A first layer coats the tablet core and comprises Opadry® II and optionally an amine compound. The active coating layer described herein is provided as a second layer coating the first layer and comprises an active pharmaceutical ingredient, Opadry® II, and an amine compound that is glycine, histidine, arginine, ethanolamine, diethanolamine or triethanolamine, or a combination thereof, where the active pharmaceutical ingredient is saxagliptin. A third layer coats the second layer and comprises Opadry® II and optionally an amine compound.

In another aspect, the present invention provides extended release tablet formulations that comprise a tablet core that comprises a disintegrant, a release modifier, a filler, a wetting agent, a lubricant, and at least one antidiabetic or a pharmaceutically acceptable salt thereof, where the antidiabetic is metformin, dapagliflozin, dapagliflozin (S) propylene glycol hydrate, or combinations thereof. A first layer coats the tablet core and comprises a coating material and optionally an amine compound. The active coating layer described herein is provided as a second layer coating the first layer and comprises an active pharmaceutical ingredient, a coating material, and an amine compound that is glycine, histidine, arginine, ethanolamine, diethanolamine or triethanolamine, or a combination thereof, where the active pharmaceutical ingredient is saxagliptin. A third layer coats the second layer and comprises a coating material and optionally an amine compound.

In another aspect, the present invention provides extended release tablet formulations that comprise a tablet core that comprises a disintegrant, a release modifier, a filler, a wetting agent, a lubricant, and at least one antidiabetic or a pharmaceutically acceptable salt thereof, where the antidiabetic is metformin, dapagliflozin, dapagliflozin (S) propylene glycol hydrate, or combinations thereof. A first layer coats the tablet core and comprises Opadry® II and optionally an amine compound. The active coating layer described herein is provided as a second layer coating the first layer and comprises an active pharmaceutical ingredient, Opadry® II, and an amine compound that is glycine, histidine, arginine, ethanolamine, diethanolamine or triethanolamine, or a combination thereof, where the active pharmaceutical ingredient is saxagliptin. A third layer coats the second layer and comprises Opadry® II and optionally an amine compound.

In another aspect, the present invention provides extended release tablet formulations that comprise a tablet core that comprises a disintegrant, a release modifier, a filler, a wetting agent, a lubricant, and at least one antidiabetic or a pharmaceutically acceptable salt thereof, where the antidiabetic is metformin, dapagliflozin, dapagliflozin (S) propylene glycol hydrate, or combinations thereof. A first layer coats the tablet core and comprises a coating material and optionally an amine compound. The active coating layer described herein is provided as a second layer coating the first layer and comprises an active pharmaceutical ingredient, a coating material, and an amine compound, where the active pharmaceutical ingredient is saxagliptin. A third layer coats the second layer and comprises a coating material and optionally an amine compound. The amine compound in each layer is as described above, for example, glycine, histidine, arginine, ethanolamine, diethanolamine or triethanolamine, or a combination thereof.

In another aspect, the present invention provides extended release tablet formulations that comprise a tablet core that comprises a disintegrant, a release modifier, a filler, a wetting agent, a lubricant, and at least one antidiabetic or a pharmaceutically acceptable salt thereof, where the antidiabetic is metformin, dapagliflozin, dapagliflozin (S) propylene glycol hydrate, or combinations thereof. A first layer coats the tablet core and comprises Opadry® II and optionally an amine compound. The active coating layer described herein is provided as a second layer coating the first layer and comprises an active pharmaceutical ingredient, Opadry® II, and an amine compound, where the active pharmaceutical ingredient is saxagliptin. A third layer coats the second layer and comprises Opadry® II and optionally an amine compound. The amine compound in each layer is as described above, for example, glycine, histidine, arginine, ethanolamine, diethanolamine or triethanolamine, or a combination thereof.

In another aspect, the present invention provides extended release tablet formulations that comprise a tablet core that comprises a disintegrant, a release modifier, a filler, a wetting agent, a lubricant, and at least one antidiabetic or a pharmaceutically acceptable salt thereof, where the antidiabetic is metformin, dapagliflozin, dapagliflozin (S) propylene glycol hydrate, or combinations thereof. A first layer coats the tablet core and comprises Opadry® II and optionally an amine compound. The active coating layer described herein is provided as a second layer coating the first layer and comprises an active pharmaceutical ingredient, Opadry® II, and an amine compound, where the active pharmaceutical ingredient is saxagliptin. A third layer coats the second layer and comprises Opadry® II and optionally an amine compound. The amine compound in each layer is glycine.

In another aspect, the present invention provides extended release tablet formulations that comprise a tablet core that comprises a disintegrant, a release modifier, a filler, a wetting agent, a lubricant, and dapagliflozin or dapagliflozin (S) propylene glycol hydrate. A first layer coats the tablet core and comprises Opadry® II white. The active coating layer described herein is provided as a second layer coating the first layer and comprises saxagliptin, Opadry®.II white, and glycine. A third layer coats the second layer and comprises Opadry® II color.

In another aspect, the present invention provides extended release tablet formulations that comprise a tablet core that comprises sodium carboxymethylcellulose, hydroxypropyl methylcellulose, microcrystalline cellulose, purified water, magnesium stearate, and dapagliflozin or dapagliflozin (S) propylene glycol hydrate. A first layer coats the tablet core and comprises Opadry® II white. The active coating layer described herein is provided as a second layer coating the first layer and comprises saxagliptin, Opadry® II white, and glycine. A third layer coats the second layer and comprises Opadry® II color.

In another aspect, the present invention provides extended release tablet formulations that comprise a tablet core that comprises a disintegrant, a release modifier, a filler, a wetting agent, a lubricant, and metformin hydrochloride. A first layer coats the tablet core and comprises Opadry®.II white. The active coating layer described herein is provided as a second layer coating the first layer and comprises saxagliptin, Opadry® II white, and glycine. A third layer coats the second layer and comprises Opadry® II color.

In another aspect, the present invention provides extended release tablet formulations that comprise a tablet core that comprises sodium carboxymethylcellulose, hydroxypropyl methylcellulose, microcrystalline cellulose, purified water, magnesium stearate, and metformin hydrochloride. A first layer coats the tablet core and comprises Opadry® II white. The active coating layer described herein is provided as a second layer coating the first layer and comprises saxagliptin, Opadry® II white, and glycine. A third layer coats the second layer and comprises Opadry® II color.

### Modified Release Materials

The present invention also contemplates the use of a modified release coating as one or more of the coating layers described herein (e.g., active coating layer, first layer, second layer, third layer). For example, the modified release coating can comprise a coating material, at least one modified release component, optionally an active pharmaceutical ingredient, and optionally an amine compound. The time release characteristics for the release of the active ingredient(s) from each or either of the regions (such as tablet core or coating) may be varied by modifying the composition of each component, including modifying any of the excipients or coatings which may be present. In particular the release of the active ingredient may be controlled by changing the composition and/or the amount of the modified release coating on the particles, if such a coating is present. If more than one modified release component is present, the modified release coating for each of these components may be the same or different. Similarly, when modified release is facilitated by the inclusion of a modified release matrix material, release of the active ingredient may be controlled by the choice and amount of modified release matrix material utilised. The modified release coating may be present, in each component, in any amount that is sufficient to yield the desired delay time for each particular component. The modified release coating may be preset, in each component, in any amount that is sufficient to yield the desired time lag between components. The lag time or delay time for the release of the active ingredient from each component may also be varied by modifying the composition of each of the components, including modifying any excipients and coatings which may be present. For example the first component may be an immediate release component wherein the active ingredient is released substantially immediately upon administration. Alternatively, the first component may be, for example, a time-delayed immediate release component in which the active ingredient is released substantially immediately after a time delay. The second component may be, for example, a time-delayed immediate release component as just described or, alternatively, a time-delayed sustained release or extended release component in which the active ingredient is released in a controlled fashion over an extended period of time.

### Suitable Components for Pharmaceutical Formulations

Binders suitable for use in the formulations of the present invention include, but are not limited to, methyl cellulose, carboxymethyl cellulose (including sodium carboxymethyl cellulose), hydroxypropyl cellulose (including HPC-SSL, HPC-SL, HPC-L, HPC-EXF, etc.), hydroxypropylmethyl cellulose, corn starch, pregelatinized starch, modified corn starch, polyvinyl pyrrolidone (PVP), hydroxypropyl methylcellulose (HPMC) (including hydroxypropyl methylcellulose 2208), lactose, gum acacia, gum arabic, gelatin, agar, ethyl cellulose, cellulose acetate, tragacanth, sodium alginate, pullulan, as well as a wax binder such as carnauba wax, paraffin, spermaceti, polyethylenes or microcrystalline wax, as well as other conventional binding agents and/or mixtures of two or more thereof. Preferred binders of the present invention are hydroxypropyl cellulose, hydroxypropyl methylcellulose, starch, and polyvinyl pyrrolidone.

Disintegrants suitable for use in the formulations of the present invention include, but are not limited to, croscarmellose sodium, crospovidone, starch, potato starch, pregelatinized starch, corn starch, sodium starch glycolate, microcrystalline cellulose, low substituted hydroxypropyl cellulose LH21, polyvinyl pyrrolidone cross linked, and other known disintegrants. Several specific types of disintegrant are suitable for use in the formulations described herein. For example, any grade of crospovidone can be used, including for example crospovidone XL-10, and includes members selected from the group consisting of Kollidon CL®, Polyplasdone XL®, Kollidon CL-M®, Polyplasdone XL-10®, and Polyplasdone INF-10®. In one embodiment, the disintegrant, if present, of the stock granulation is sodium starch glycolate, croscarmellose sodium and/or crospovidone. The preferred disintegrants are croscarmellose sodium, crospovidone, starch, and sodium starch glycolate.

Fillers suitable for use in the formulations of the present invention include, but are not limited to, cellulose derivatives, such as microcrystalline cellulose or wood cellulose (including microcrystalline cellulose 302), lactose, lactose anhydrous, sucrose, starch, pregelatinized starch, dextrose, mannitol (including mannitol Pearlitol SD 200), fructose, xylitol, sorbitol, corn starch, modified corn starch, inorganic salts such as calcium carbonate, calcium phosphate, dicalcium phosphate, calcium sulfate, dextrin/dextrates, maltodextrin, compressible sugars, and other known bulking agents or fillers, and/or mixtures of two or more thereof. Several types of microcrystalline cellulose are suitable for use in the formulations described herein, for example, microcrystalline cellulose selected from the group consisting of Avicel® types: PH101, PH102, PH103, PH105, PH 112, PH113, PH200, PH301, and other types of microcrystalline cellulose, such as silicified microcrystalline cellulose. Several types of lactose are suitable for use in the formulations described herein, for example, lactose selected from the group consisting of anhydrous lactose, lactose monohydrate, lactose fast flow, directly compressible anhydrous lactose, and modified lactose monohydrate. The preferred fillers of the present invention are microcrystalline cellulose and lactose monohydrate.

Glidants and/or anti-adherents suitable for use in the formulations of the present invention include, but are not limited to, silicon dioxide, colloidal silicon dioxide, magnesium silicate, magnesium trisilicate, talc, and other forms of silicon dioxide, such as aggregated silicates and hydrated silica. Preferred glidants are talc, lecithin, and colloidal silicon dioxide.

Lubricants suitable for use in the formulations of the present invention include, but are not limited to, magnesium stearate, zinc stearate, calcium stearate, talc, carnauba wax, stearic acid, palmitic acid, sodium stearyl fumarate sodium laurel sulfate, glyceryl palmitostearate, palmitic acid, myristic acid and hydrogenated vegetable oils and fats, as well as other known lubricants, and/or mixtures of two or more thereof. The preferred lubricants of the present invention are magnesium stearate and stearic acid.

Release modifiers suitable for use in the formulations of the present invention include, but are not limited to, sodium alginate, hydroxypropyl methylcellulose (HPMC), hydroxypropyl cellulose (HPC), methyl cellulose (MC), ethyl cellulose (EC), acrylate polymers, any grades of Eudragit® such as Eudragit RL or RS, poly acrylic acid and poly acrylate and methacrylate copolymers such as those sold under the Trade Mark Eudragite S and L, polyvinyl acetaldiethylamino acetate, hydroxypropyl methylcellulose acetate succinate, shellac; hydrogels and gel-forming materials, such as carboxyvinyl polymers, sodium alginate, sodium carmellose, calcium carmellose, sodium carboxymethyl starch, poly vinyl alcohol, hydroxyethyl cellulose, methyl cellulose, gelatin, starch, and cellulose based cross-linked polymers-in which the degree of crosslinking is low so as to facilitate adsorption of water and expansion of the polymer matrix, hydoxypropyl cellulose, hydroxypropyl methylcellulose, polyvinylpyrrolidone, crosslinked starch, microcrystalline cellulose, chitin, aminoacryl-methacrylate copolymer (Eudragit® RS-PM, Rohm & Haas), pullulan, collagen, casein, agar, gum arabic, sodium carboxymethyl cellulose, (swellable hydrophilic polymers) poly(hydroxyalkyl methacrylate) (mol. wt. ∼5k g/mol-5,000k g/mol), polyvinylpyrrolidone (mol. wt. ∼10k g/mol -360k g/mol), anionic and cationic hydrogels, polyvinyl alcohol having a low acetate residual, a swellable mixture of agar and carboxymethyl cellulose, copolymers of maleic anhydride and styrene, ethylene, propylene or isobutylene, pectin (mol. wt. ∼30k g/mol - 300k g/mol), polysaccharides such as agar, acacia, karaya, tragacanth, algins and guar, polyacrylamides, Polyox® polyethylene oxides (mol. wt. ∼100k g/mol - 5,000k g/mol), AquaKeep® acrylate polymers, diesters of polyglucan, crosslinked polyvinyl alcohol and poly N-vinyl-2-pyrrolidone, sodium starch glucolate (e.g. Explotab®; Edward Mandell C. Ltd.); hydrophilic polymers such as polysaccharides, methyl cellulose, sodium or calcium carboxymethyl cellulose, hydroxypropyl methyl cellulose, hydroxypropyl cellulose, hydroxyethyl cellulose, nitro cellulose, carboxymethyl cellulose, cellulose ethers, polyethylene oxides (e.g. Polyox®, Union Carbide), methyl ethyl cellulose, ethylhydroxy ethylcellulose, cellulose acetate, cellulose butyrate, cellulose propionate, gelatin, collagen, starch, maltodextrin, pullulan, polyvinyl pyrrolidone, polyvinyl alcohol, polyvinyl acetate, glycerol fatty acid esters, polyacrylamide, polyacrylic acid, copolymers of methacrylic acid or methacrylic acid (e.g. Eudragit®, Rohm and Haas), other acrylic acid derivatives, sorbitan esters, natural gums, lecithins, pectin, alginates, ammonia alginate, sodium, calcium, potassium alginates, propylene glycol alginate, agar, and gums such as arabic, karaya, locust bean, tragacanth, carrageens, guar, xanthan, scleroglucan and mixtures and blends thereof.

Wetting agents suitable for use in the formulations of the present invention include, but are not limited to, polysorbate, polyvinylpyrrolidone, anionic surfactants (based on permanent anions such as sulfate, sulfonate, phosphate or pH-dependent anions such as carboxylate) such as ammonium lauryl sulfate, sodium lauryl sulfate (SDS), sodium laureth sulfate (also known as sodium lauryl ether sulfate (SLES)), sodium myreth sulfate; dioctyl sodium sulfosuccinate, perfluorooctanesulfonate (PFOS), perfluorobutanesulfonate; alkyl benzene sulfonates; alkyl aryl ether phosphates, alkyl ether phosphates, alkyl carboxylates such as fatty acid salts (soaps), sodium stearate, sodium lauroyl sarcosinate; carboxylate fluorosurfactants: perfluorononanoate, perfluorooctanoate; cationic surfactants that are pH-dependent (e.g., ,primary, secondary or tertiary amines) such as octenidine dihydrochloride; permanently charged quaternary ammonium cation such as alkyltrimethylammonium salts, e.g., cetyl trimethylammonium bromide (CTAB) or hexadecyl trimethyl ammonium bromide, cetyl trimethylammonium chloride (CTAC); cetylpyridinium chloride (CPC); polyethoxylated tallow amine (POEA); benzalkonium chloride (BAC); benzethonium chloride (BZT); 5-Bromo-5-nitro-1,3-dioxane; dimethyldioctadecylammonium chloride; dioctadecyldimethylammonium bromide (DODAB); zwitterionic or amphoteric surfactants such as sulfonates, e.g., CHAPS (3-[(3-Cholamidopropyl)dimethylammonio]-1-propanesulfonate), sultaines: cocamidopropyl hydroxysultaine; carboxylates, such as amino acids, imino acids, betaines, e.g., cocamidopropyl betaine; phosphates such as lecithin; nonionic surfactants such as fatty alcohols, e.g., cetyl alcohol, stearyl alcohol, cetostearyl alcohol (consisting predominantly of cetyl and stearyl alcohols), oleyl alcohol; polyoxyethylene glycol alkyl ethers (Brij): CH₃-(CH₂)₁₀₋₁₆-(O-C₂H₄)₁₋₂₅-OH: octaethylene glycol monododecyl ether, pentaethylene glycol monododecyl ether; polyoxypropylene glycol alkyl ethers: CH₃-(CH₂)₁₀₋₁₆-(O-C₃H₆)₁₋₂₅-OH; glucoside alkyl ethers: CH₃-(CH₂)₁₀₋₁₆-(O-Glucoside)₁₋₃-OH: decyl glucoside, lauryl glucoside, octyl glucoside; polyoxyethylene glycol octylphenol ethers: C₈H₁₇-(C₆H₄)-(O-C₂H₄)₁₋₂₅-OH: triton X-100; polyoxyethylene glycol alkylphenol ethers: C₉H₁₉-(c₆H₄)-(O-C₂H₄)₁₋₂₅-OH: oonoxynol-9; glycerol alkyl esters such as glyceryl laurate; polyoxyethylene glycol sorbitan alkyl esters: polysorbates; sorbitan alkyl esters: spans; cocamide MEA, cocamide DEA; dodecyl dimethylamine oxide; and block copolymers of polyethylene glycol and polypropylene glycol, such as poloxamers.

In certain embodiments, one or more of the coating materials described herein have the propensity to form, or support the formation of, formylating species including, but not limited to, formic acid, formates, formaldehyde, and/or derivatives thereof. For example, in one embodiment, at least the coating material of an active coating layer has the propensity to form, or support the formation of, formylating species including, but not limited to, formic acid, formates, formaldehyde, and/or derivatives thereof.

The term "Opadry® II color" includes, but is not limited to, Opadry® HP, Opadry® II white, Opadry® II orange, Opadry® II brown, or Opadry® II yellow. In addition to Opadry II color, the third layer can also optionally include an anti-adherent or glidant such as talc, fumed silica, or magnesium stearate, or an opacifying agent, such as titanium dioxide. The third layer may optionally include combinations of one or more Opadry® II color materials. Preferred coatings of the present invention are Opadry® II white, Opadry® II brown, Opadry® II orange, and Opadry® II yellow.

The term "Opadry® HP" as used here means a film coating for a tablet that comprises 40% polyvinyl alcohol, 20% polyethylene glycol, 15% talc, and 25% titanium dioxide.

The term "Opadry® II" as used here means a film coating for a tablet that comprises polyvinyl alcohol, titanium dioxide, polyethylene glycol (PEG), and talc. Opadry® II white 85F 18422 is comprised of polyvinyl alcohol, titanium dioxide, polyethylene glycol, and talc. Opadry® II Yellow 85F92582 is comprised of polyvinyl alcohol, titanium dioxide, polyethylene glycol, talc, and yellow iron dioxide.

The term "pharmaceutically acceptable salt" or "salt," as used herein, refers to salts that are well known in the art. For example, S. M Berge et al. describe pharmaceutically acceptable salts in detail in J. Pharmaceutical Sciences, 66:1-19 (1977). Examples of pharmaceutically acceptable salts include acetic acid, aspartic acid, benzenesulfonic acid, benzoic acid, butyric acid, citric acid, fumaric acid, hydrochloric acid, hydrobromic acid, lactic acid, maleic acid, malonic acid, methanesulfonic acid, 4-methylbenzenesulfonic acid, nicotinic acid, phosphoric acid, succinic acid, sulfuric acid, or tartaric acid, prepared by using methods well known in the art. The preferred pharmaceutically acceptable salt of saxagliptin for use in the methods of the present invention is HCl.

The term "water soluble antioxidant" as used herein means any antioxidant with a water solubility equal to, or greater than, 0.1 mg/mL.

Suitable anti-diabetic agents for use in the formulations of the present invention include, but are not limited to, alpha glucosidase inhibitors (acarbose or miglitol), insulins (including insulin secretagogues or insulin sensitizers), meglitinides (repaglinide, nateglinide, or KAD1229 (PF/Kissei)), sulfonylureas, biguanides (buformin, metformin, phenformin), biguanide/glyburide combinations (Glucovance®), thiazolidinediones, PPAR-alpha agonists, PPAR-gamma agonists, PPAR alpha/gamma dual agonists, glycogen phosphorylase inhibitors, inhibitors of fatty acid binding protein (aP2), GPR-119 modulators, GPR 40 modulators, glucokinase inhibitors, glucagon-like peptide-1 (GLP-1) and other agonists of the GLP-1 receptor, SGLT2 inhibitors, and dipeptidyl peptidase IV (DPP4) inhibitors other than saxagliptin. The antidiabetic agents used in the formulations of the present invention can be used in the amounts indicated in the Physician's Desk Reference or as in the cited patents and patent applications set out above or as otherwise known and used by one of ordinary skill in the art.

Suitable thiazolidinediones include, but are not limited to, rosiglitazone, pioglitazone, troglitazone, MCC-555 (disclosed in U.S. Patent No. 5,594,016, Mitsubishi), faraglitazar (GI-262570, Glaxo-Wellcome), englitazone (CP-68722, Pfizer) or darglitazone (CP-86325, Pfizer; isaglitazone, MIT/Johnson& Johnson), reglitazar (JTT-501, (JPNT/Pharmacia & Upjohn), rivoglitazone (R-119702, Sankyo/WL), liraglutide (NN-2344, Dr. Reddy/NN), and (Z)-1,4-bis-4-[(3,5-dioxo-1,2,4-oxadiazolidin-2-yl-methyl)]phenoxybut-2-ene (YM-440, Yamanouchi). The preferred thiazolidinediones are rosiglitazone and pioglitazone.

Suitable sulfonylureas include, but are not limited to, acetohexamide, chlorpropamide, glibenclamide (glyburide), gliclazide, glimepiride, glipizide, glyclopyramide, tolazamide, and tolbutamide. The preferred sulfonylurea is glyburide.

Suitable forms of the antidiabetic agent metformin for use in the present invention's formulations include pharmaceutically acceptable salts thereof such as the hydrochloride, hydrobromide, fumarate, succinate, p-chlorophenoxy acetate or embonate. The fumarate and succinate salts are preferably metformin (2:1) fumarate, and metformin (2:1) succinate. Metformin hydrochloride is the preferred salt.

Suitable PPAR-alpha agonists, PPAR-gamma agonists and PPAR alpha/gamma dual agonists include, but are not limited to, muraglitazar, peliglitazar, tesaglitazar AR-HO39242 (Astra/Zeneca), GW-501516 (Glaxo-Wellcome), KRP297 (Kyorin Merck), as well as those disclosed by Murakami et al, "A Novel Insulin Sensitizer Acts As a Coligand for Peroxisome Proliferation - Activated Receptor Alpha (PPAR alpha) and PPAR gamma. Effect on PPAR alpha Activation on Abnormal Lipid Metabolism in Liver of Zucker Fatty Rats", Diabetes 47, 1841-1847 (1998); International Patent Application Publication no. WO 01/21602 and in U.S. Patent No. 6,414,002 and U.S Patent No. 6,653,314 employing dosages as set out therein. In one embodiment, the compounds designated as preferred in the cited references are preferred for use herein.

Suitable aP2 inhibitors include, but are not limited to, those disclosed in U.S. Patent Application Serial No. 09/391,053, filed September 7, 1999 (issued as U.S. Patent no. 7,390,824), and in U.S. Patent No. 6,548,529 employing dosages as set out therein.

Suitable DPP4 inhibitors include saxagliptin, sitagliptin, vildagliptin, linagliptin, dutogliptin, and alogliptin, as well as those disclosed in International Patent Applications nos. WO99/38501, WO99/46272, WO99/67279 (PROBIODRUG), WO99/67278 (PROBIODRUG) and WO99/61431 (PROBIODRUG); NVP-DPP728A (1-[[[2-[(5-cyanopyridin-2-yl)amino]ethyl]amino]acetyl]-2-cyano-(S)-pyrrolidine) (Novartis) as disclosed by Hughes et al, Biochemistry, 38(36), 11597-11603, 1999; TSL-225 (tryptophyl-1,2,3,4-tetrahydroisoquinoline-3-carboxylic acid (disclosed by Yamada et al, Bioorg. & Med. Chem. Lett. 8 (1998) 1537-1540); 2-cyanopyrrolidides and 4-cyanopyrrolidides, as disclosed by Ashworth et al, Bioorg. & Med. Chem. Lett., Vol. 6, No. 22, pp 1163-1166 and 2745-2748 (1996); and the compounds disclosed in U.S. Patent Application Serial No. 10/899,641 (published as U.S. Patent Application Publication no. 2005/0038020) employing dosages as set out in the above references. Preferred DPP4 inhibitors for the tablet core (anti-diabetic agent) are selected from sitagliptin and vildagliptin. Saxagliptin is the preferred active pharmaceutical ingredient for the coatings or layers.

Saxagliptin or (1*S*,3*S*,5*S*)-2-[(2*S*)-2-amino-2-(3-hydroxyadamantan-1-yl)acetyl]-2-azabicyclo[3.1.0]hexane-3-carbonitrile can be prepared using the synthetic procedures described in U.S. Patent no. 6,395,767, in particular Example 60. The present invention encompasses the use of pharmaceutically acceptable salts in the formulations, as defined herein, of saxagliptin, in particular the benzoic acid, fumaric acid, hydrobromic acid, hydrochloric acid, methane sulfonic acid, tartaric acid, trifluoroacetic acid, salts of saxagliptin, for use in the methods described herein. Saxagliptin salts are prepared using techniques well known in the art or by using the procedures described in U.S. Patents nos. 6,395,767 and 7,420,079, and International Patent Application Publication no. WO 08/131149. The present invention also encompasses the use crystalline forms of saxagliptin as hydrates and/or solvates in the formulations. In particular, the use in the formulations of the invention of the mono hydrate of saxagliptin is encompassed. Other hydrate forms are also contemplated by the present invention including the hemi hydrate or (2:1) saxagliptin:H₂O. Hydrates of saxagliptin salts are also contemplated in the formulations of the present invention and include the crystalline salt/hydrates disclosed in International Patent Application Publication no. WO 08/131149. Saxagliptin inhibits DPP4, Kᵢ=1.3 nM in Human DPP4 Inhibition.

Suitable SGLT2 inhibitors include canagliflozin, dapagliflozin, remogliflozin, and sergliflozin.

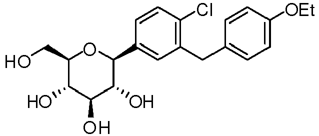

### Dapagliflozin

### (2S,3R,4R,5S,6R)-2-(4-chloro-3-(4-ethoxybenzyl)phenyl)-6-(hydroxymethyl)tetrahydro-2H-pyran-3,4,5-triol

Dapagliflozin can be prepared using similar procedures as described in U.S. Patent No. 6,515,117 or International Patent Application Publications nos. WO 03/099836 and WO 2008/116179. SGLT2 EC₅₀ = 1.1 nM.

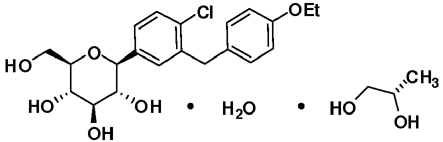

### Dapagliflozin (S) PGS

### (2S,3R,4R,5S,6R)-2-(4-chloro-3-(4-ethoxybenzyl)phenyl)-6-(hydroxymethyl)tetrahydro-2H-pyran-3,4,5-triol (S)-propane-1,2-diol hydrate (1:1:1)

Dapagliflozin (S) propylene glycol hydrate (1:1:1) can be prepared using similar procedures as described in International Patent Application Publications nos. WO 08/002824 and WO 2008/116179. SGLT2 EC₅₀ = 1.1 nM.

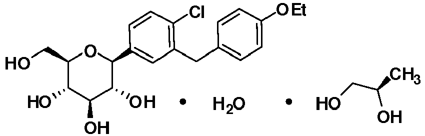

### Dapagliflozin (R) PGS

### (2S,3R,4R,5S,6R)-2-(4-chloro-3-(4-ethoxybenzyl)phenyl)-6-(hydroxymethyl)tetrahydro-2H-pyran-3,4,5-triol (R)-propane-1,2-diol hydrate (1:1:1)

Dapagliflozin (R) propylene glycol hydrate (1:1:1) can be prepared using similar procedures as described in International Patent Application Publications nos. WO 08/002824 and WO 2008/116179. SGLT2 EC₅₀ = 1.1 nM.

The present invention also contemplates formulations where the SGLT2 inhibitor is a compound of Formula (I) as described in U.S Patent No. 6,414,126. Other SGLT2 inhibitors contemplated by the present disclosure include sergliflozin, remogliflozin, etabonate, canagliflozin, BI-10773 and BI-44847, ASP-1941, R-7201, LX-4211, YM-543, AVE 2268, TS-033 or SGL-0100, and the compounds disclosed in U.S. Patents nos. 7,589,193 and 7,288,528, International Patent Application Publications nos. WO 2007/007628 and WO 2009/03596, European Patent Application Publication no. EP 2 009 010, and U.S. Patent Application Publications nos. US 2009/0030198, US and US 2007/0197623. The following SGLT2 inhibitors, in addition to dapagliflozin, are preferred: 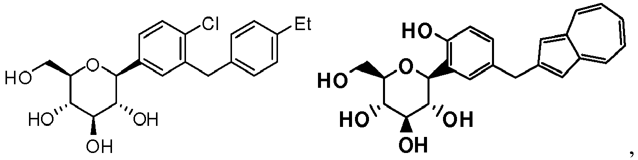 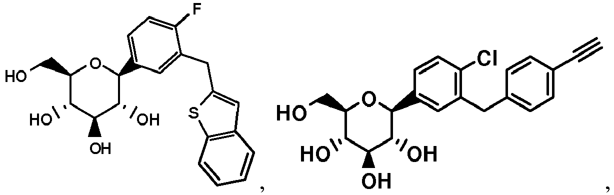 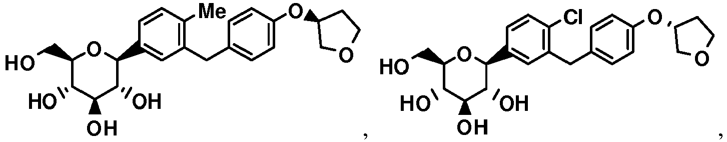 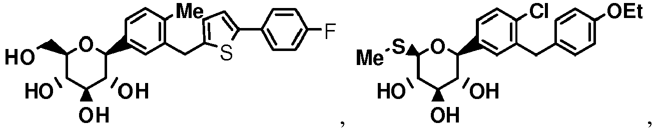 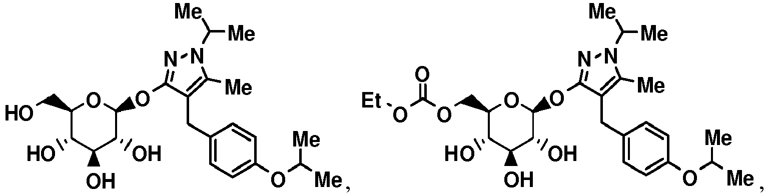 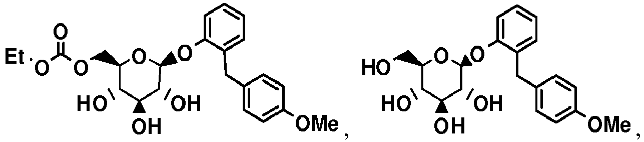 and

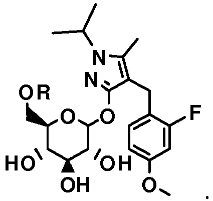

### Examples

### Example 1

Saxagliptin tablets 2.5 mg were exposed for one day to 60°C/75% RH and then stored at 30°C/65% RH for 12 months in HDPE bottles containing desiccant and induction sealed. The tablets had the formulation provided in Table 1:

**Table 1**

| **Material** | **Function** | **Quantity in Mg/tablet** |
|---|---|---|
| **Core tablet composition:** | | |
| Lactose monohydrate | Filler | 99 |
| Microcrystalline cellulose | Filler | 90 |
| Croscarmellose sodium | Disintegrant | 10 |
| Magnesium Stearate | Lubricant | 1 |

| **First Layer Composition:** | | |
|---|---|---|
| Opadry® II White | Coating Material | 21 |

| **Second Layer Composition:** | | |
|---|---|---|
| Saxagliptin | API | 2.5 |
| Opadry® II White | Coating Material | 20 |

| **Third Layer Composition:** | | |
|---|---|---|
| Opadry II color | Coating Material | 17 |
| | | |
| Hydrochloric Acid Solution, IN | For pH adjustment | q.s. |
| Purified Water | Vehicle for coating suspension | q.s. |
| Sodium Hydroxide Solution, IN | For pH adjustment | q.s. |
| Opacode | Printing Ink | 0.03 |

In a control study, saxagliptin tablets were stored in HDPE bottles at 30°C/65% RH for 12 months without prior exposure to 60°C/75% RH.

The NFA level in the exposed tablets was significantly higher than the unexposed tablets (∼ 0.4 - 0.5% w/w of saxagliptin versus < 0.05% w/w). The level of CA in both cases was similar (∼ 0.05% w/w of saxagliptin).

### Example 2

Four batches of Opadry® II white were used to prepare coating suspensions at 17.5% w/w concentration in water and adjusted to pH 2.0 ± 0.3 using hydrochloric acid, as per the manufacturing process of the saxagliptin second layer coating suspension. The suspensions were stored at room temperature (∼25°C) for 8 or 14 days. They were analyzed for total formyl species level by a derivatization gas chromatography (GC) method. The results are shown in Table 2.

**Table 2**

| **Formic Acid Level in Opadry Suspension Stored at Room Temperature** | | | |
|---|---|---|---|
| **Batch Number of Opadry® II White** | **Level of Formic Acid (ppm based on mass of Opadry Powder)** | | |
| | **Initial** | **Day 8** | **Day 14** |
| 1 | 112 | 427 | 582 |
| 2 | 130 | 627 | 960 |
| 3 | 50 | 225 | 339 |
| 4 | 132 | 305 | 446 |

The data in Table 2 shows a significant increase in formic acid levels in the suspensions of the coating material for all of the Opadry® II white batches. Interpolation of this data to 72 hours of hold time storage, which is recommended by the vendor and usually followed in a drug product manufacturing operation, indicates a 1-2 fold increase in formic acid levels.

### Example 3

### Effect of Holding of Opadry Suspension on NFA Formation in Tablets.

Opadry® II white powder was dispersed in water at 17.5% w/w concentration in water and adjusted to pH 2.0 ± 0.3 using hydrochloric acid, as per the manufacturing process of saxagliptin second layer coating suspension. This suspension was used to prepare saxagliptin tablets 2.5 mg using a three layer coating procedure (described, for example, in U.S. Patent Application Publication no. 2005/0266080), while holding the suspension for up to 72 hours (maximum hold time permitted during routine manufacturing) for each layer in one case and up to 12 hours (minimum possible during routine manufacturing) in another case. In the former case, the suspension was held for 48 hours before the initiation of coating, resulting in the total storage time of suspension (until the end of coating) of up to 72 hours. In the latter case, coating was initiated immediately after the preparation of suspension, resulting in the total storage time of suspension (until the end of coating) of up to 12 hours. The coated tablets were packaged in HDPE bottles with desiccants and induction sealed. The packaged drug product was kept at 40°C and 75% RH for 6 months or at 50°C for 1 month, followed by impurity analysis. The results of this study are shown in Table 3.

**Table 3**

| **Impurity Levels in Saxagliptin Tablets 2.5 mg with Holding of Opadry® Suspension at Room Temperature for Different Periods of Time During Product Manufacturing** | | | |
|---|---|---|---|
| **Impurity (% w/w of saxagliptin)** | **Storage condition in HDPE packs** | **Holding time of suspension (hours)** | |
| | | **12** | **72** |
| **% NFA** | **Initial** | < 0.05 | < 0.05 |
| | **40°C/75% RH/6 months** | 0.06 | **0.24** |
| | **50°C/1 month** | < 0.05 | **4.16** |
| **% CA** | **Initial** | < 0.05 | 0.08 |
| | **40°C/75% RH/6 months** | 0.09 | 0.19 |
| | **50°C/1 month** | < 0.05 | 0.26 |
| **Total** | **Initial** | 0.06 | 0.08 |
| | **40°C/75% RH/6 months** | 0.28 | 0.49 |
| | **50°C/1 month** | 0.07 | 5.31 |

After storage at 40°C/75% RH for 6 months, the NFA level in drug product that was stored for 72 hours at room temperature was found to be 0.24% w/w of saxagliptin, while it was 0.06% w/w for the drug product stored for 12 hours during product manufacturing. Similarly, after storage at 50°C for 1 month, the NFA level in drug product that was stored for 72 hours was found to be 4.16% w/w of saxagliptin, and <0.05% w/w for the drug product stored for 12 hours during product manufacturing.

In comparing the % CA and total impurities among batches with different hold times, NFA was found to be the major degradant that increased as a function of hold time of suspension. These data indicated that holding time of suspension during product manufacturing had an impact on the growth of NFA during drug product stability.

### Example 4

### Effectiveness of Amine Compounds in Reducing Formation of NFA of Saxagliptin in Opadry® Films

Opadry® II white powder was dispersed in water in an aqueous solution of saxagliptin at 6% w/w total concentration (mass ratio of Opadry to saxagliptin = 1:9). About 0.5 mL of the suspension was transferred to each of four 5 mL lyophilization vials. To three of the vials were respectively added ethanolamine, diethanolamine and triethanolamine, to provide amine in the dispersion at concentrations of 2% w/w; the fourth was used as a control. The open vials were allowed to incubate overnight at 40 °C to form films on the bottom inside surfaces of the vials. The vials were sealed and incubated at 60 °C for five days. The films were analyzed by HPLC for to determine the amount of NFA-saxagliptin in each film. The results are provided in Table 4.

**Table 4**

| **N-Formyl Adduct of Saxagliptin in Saxagliptin/Opadry® II Films** | |
|---|---|
| **Amine Compound** | **% NFA (as a % of saxagliptin peak)** |
| None (control) | 2.63 |
| Ethanolamine | 0 |
| Diethanolamine | 3.61 |
| Triethanolamine | 1.01 |

### Example 5

### Effectiveness of Amine Compounds in Reducing Formation of NFA of Saxagliptin Hydrochloride in Opadry® Films

The experiments of Example 4 were repeated, with the addition of 1 N hydrochloric acid to the solution in amount equimolar with the saxagliptin. The films were analyzed by HPLC for to determine the amount of NFA-saxagliptin in each film. The results are provided in Table 5.

**Table 5**

| **N-Formyl Adduct of Saxagliptin in Saxagliptin Hydrochloride/Opadry® II Films** | |
|---|---|
| **Amine Compound** | **% NFA (as a % of saxagliptin peak)** |
| None (control) | 2.73 |
| Ethanolamine | 0 |
| Diethanolamine | 0.62 |
| Triethanolamine | 1.83 |

In the experiments of Examples 3 and 4, the primary amines were the most effective in preventing NFA formation. Without intending to the bound by theory, the inventors surmise that the amine compound acts as a scavenger of any formyl species in the coating material, thus depleting the amount of formyl species available to react with a primary or secondary amine drug product such as saxagliptin. Primary amines would generally be more reactive with formyl species than would secondary or tertiary amines, as primary amines are more nucleophilic and less hindered. The results of Examples 3 and 4 suggest that primary amines can effectively compete with amine-containing drug products such as saxagliptin for the available formyl species, and can therefore prevent the formation of NFAs.

### Example 6

### Effectiveness of Amino Acid Compounds in Reducing Formation of NFA of Saxagliptin in Opadry® Films

Opadry® II white powder was dispersed in water at 6% w/w solids in an aqueous solution of saxagliptin saxagliptin at 6% w/w total concentration (mass ratio of Opadry to saxagliptin = 1:9). About 0.5 mL of the suspension was transferred to each of five 5 mL lyophilization vials. To four of the vials were respectively added glycine, histidine, lysine and arginine, to provide amino acid in the dispersion at concentrations of 2% w/w; the fourth was used as a control. The open vials were allowed to incubate overnight at 40 °C to form films on the bottom inside surfaces of the vials. The vials were sealed and incubated at 60 °C for five days. The films were analyzed by HPLC for to determine the amount of NFA-saxagliptin in each film. The results are provided in Table 6.

**Table 6**

| **N-Formyl Adduct of Saxagliptin in Saxagliptin/Opadry® II Films** | | | |
|---|---|---|---|
| **Amine Compound** | **% NFA (as a % of saxagliptin peak)** | **pKa of the -amino group** | **pKa of the side chain amine** |
| None (control) | 2.63 | --- | --- |
| Glycine | 0 | 9.60 | --- |
| Histidine | 1.68 | 9.17 | 6.04 |
| Lysine | 2.84 | 8.95 | 10.79 |
| Arginine | 0 | 9.04 | 12.48 |

### Example 7

### Effectiveness of Amine Compounds in Reducing Formation of NFA of Saxagliptin Hydrochloride in Opadry® Films

The experiments of Example 6 were repeated, with the addition of 1 N hydrochloric acid to the solution in amount equimolar with the saxagliptin. The films were analyzed by HPLC for to determine the amount of NFA-saxagliptin in each film. The results are provided in Table 7.

**Table 7**

| **N-Formyl Adduct of Saxagliptin in Saxagliptin Hydrochloride/Opadry® II Films** | | | |
|---|---|---|---|
| **Amine Compound** | **% NFA (as a % of saxagliptin peak)** | **pKa of the -amino group** | **pKa of the side chain amine** |
| None (control) | 2.73 | --- | --- |
| Glycine | 0 | 9.60 | --- |
| Histidine | n.a. | 9.17 | 6.04 |
| Lysine | 2.08 | 8.95 | 10.79 |
| Arginine | 0 | 9.04 | 12.48 |

In the experiments of Examples 5 and 6, it was expected that the amino acids bearing two amines would be more powerful formyl species scavengers than would glycine, with the rank order of protection following the basicity of the side chain amine group. Unexpectedly, it was found that glycine, an amino acid with only one amine, was the most potent at protecting saxagliptin from reaction with formyl species. Moreover, the use of other amino acids, including arginine, also resulted in the formation of extraneous unidentified peaks in the chromatogram. Accordingly, while amino acids other than glycine can be used in practicing the present invention, glycine is a surprisingly preferred amino acid.

### Example 8

### Effectiveness of Glycine in Reducing Formation of NFA in Saxagliptin/Metformin Hydrochloride Dosage Form

To confirm the stabilizing effect of glycine in a drug product, a batch of saxagliptin/metformin immediate release 2.5/1000 tablets was manufactured. The tablets are prepared by coating a Met IR core tablet with PVA-based Opadry coating material in a three layer coating process to embed saxagliptin in the coating material. Other fixed dose combination products can be prepared by modifying the API loading and composition of the excipients in the core or the coating of the tablet and total tablet weight. Table 8 provides the amounts of the various components in the tablet.

**Table 8**

| **Composition of Saxa/Met IR Tablet** | |
|---|---|
| **Material** | **Quantity (mg/tablet)** |
| **Core tablet composition:** | |
| Metformin HCl | 500 |
| Magnesium stearate | 2.8 |
| Povidone | 20.0 |
| Purified water ca. | 5.2 |
| | |

| **First layer composition:** | |
|---|---|
| Opadry® II White | 21.2 |
| | |

| **Second layer composition:** | |
|---|---|
| Saxagliptin | 2.5 |
| Opadry® II White | 20.0 |
| Glycine | 5.9 |
| | |

| **Third layer composition:** | |
|---|---|
| Opadry® II Pink | 17.2 |
| | |
| 1N RCl | q.s. for pH adjustment |
| 1N NaOH | q.s. for pH adjustment |
| Purified water | q.s. as vehicle for coating suspensions |
| Opacode® | 0.03 |

To prepare the second layer composition, saxagliptin was dissolved in water and pH adjusted to 2.0 ± 0.3 using 1 N HCl. Glycine hydrochloride was added, then Opadry II white was suspended. The pH of the final suspension was adjusted to pH 2.0, using 1 N HCl or 1 N NaOH as necessary. The use of glycine in the second layer composition did not result in any major changes in suspension characteristics, processing issues, or coating defects, as compared to similar compositions without glycine.

A batch of control tablets without glycine was also prepared.

The tablets were incubated at 40 °C/75% relative humidity in open dish conditions for 14 days. This storage condition has been identified as a surrogate for long term stability. The results of HPLC analysis for impurities are provided in Table 9.

**Table 9**

| **Impurities in Saxa/Met IR Tablets With and Without Glycine in Active Layer** | | | |
|---|---|---|---|
| **Amine Compound** | **Component/Impurity** | **pKa of the - amino group** | **pKa of the side chain amine** |
| None (control) | Saxagliptin | 91.58 | 95.46 |
| Glycine | NFA | 3.88 | 0.14 |
| Histidine | CA | 3.53 | 3.99 |
| Lysine | DKP | 0.27 | 0.24 |
| Arginine | All other | 0.74 | 0.17 |

### Example 9

### Coating Formulation

A typical coating formulation for film coating of oral solid dosage forms, such as an immediate release tablet, is exemplified in Table 10. An active pharmaceutical ingredient that is a primary or secondary amine susceptible to N-formylation can be included to provide an active coating.

**Table 10**

| **Typical Coating Formulation for Film Coating a Tablet** | | | |
|---|---|---|---|
| **Functional category of the component** | **Examples of components** | **Range (% w/w of total coating material)** | **Preferred Range (% w/w of total coating material)** |
| Polymer | Hydroxypropyl methylcellulose (HPMC), polyvinyl alcohol (PVA), ethyl cellulose (EC), methyl cellulose (MC), hydroxypropyl cellulose (HPC), acrylate polymers (e.g., Eudragits) | 25 - 55 | 35 - 45 |
| Plasticizer | Polyethylene glycol (PEG), glycerin, castor oil, propyl gallate (PG), surfactants | 1 - 50 | 20 - 30 |
| Opacifier | Titanium dioxide, pigment, aluminum lakes, iron oxides, natural colors | 0 - 40 | 10 - 30 |
| Glidant | Talc, lecithin | 0 - 15 | 5 - 15 |
| Amine Compound | Glycine, lysine, histidine, arginine, ethanolamine, diethanolamine or triethanolamine or a combination thereof | so as to result in a molar ratio of amine to active ingredient in the range of 1:1 to 20:1 | so as to result in a molar ratio of amine to active ingredient in the range of 1:1 to 10:1 |

### Example 10

### Tablet Formulation with Drug in the Core

The findings disclosed in this invention are also applicable to oral solid dosage forms with drug in the core of the tablet. The formulations of this invention can be used for drugs that react with formic acid or a formylating species, such as formic acid esters or anhydrides. Amine compounds may be added to formulations that contain residual levels of formic acid or a different formylating species (e.g., in the range of 5 ppm or higher). The formulations may contain a formylating species initially and/or formylating species may be formed on storage at high temperature (such as 40 °C to 60 °C) and/or humidity (such as 75% RH to 95% RH) conditions over a period of 2 days to 3 months. This tablet formulation may or may not be coated. If a tablet is coated, the amine compound described herein can be used either in the coating or in the core of the tablet or both.

An example of a coated immediate release tablet formulation is listed in Table 11 (without listing the amine compound). Based on the findings of this invention, a proposed formulation composition for the example cited in Table 11 would further include an amine compound, as described herein. This amine compound can be incorporated in the core of the tablet (Table 12), the coating material (Table 13) or both (Table 14).

**Table 11**

| **Typical Composition of an Immediate Release Tablet Formulation** | | | | |
|---|---|---|---|---|
| **Location in tablet** | **Functional class** | **Examples of components** | **Range*** | **Preferred Range*** |
| Core | Active pharmaceutical ingredient | Saxagliptin, sitagliptin, metformin | 1 - 98 | 5 - 65 |
| | Filler | Lactose monohydrate, microcrystalline cellulose | 5 - 95 | 20 - 80 |
| | Binder | Hydroxypropyl cellulose (HPC), polyvinyl pyrrolidone (PVP), starch, hydroxypropyl methylcellulose (HPMC) | 1 - 20 | 2 - 12 |
| | Disintegrant | Croscarmellose sodium, crospovidone, starch, sodium starch glycollate | 1 - 20 | 2 - 12 |
| | Glidant | Talc, colloidal silicon dioxide | 0.1 - 10 | 0.5 - 7.5 |
| | Lubricant | Magnesium stearate, stearic acid, | 0.1 - 10 | 0.5 - 2.5 |
| Coating | Polymer | Hydroxypropyl methylcellulose (HPMC), polyvinyl alcohol (PVA), ethyl cellulose (EC), methyl cellulose (MC), hydroxypropyl cellulose (HPC), acrylate polymers (e.g., Eudragits) | 25 - 55 | 35 - 45 |
| | Plasticizer | Polyethylene glycol (PEG), glycerin, castor oil, propyl gallate (PG), surfactants | 1 - 50 | 20 - 30 |
| | Opacifier | Titanium dioxide, pigment, aluminum lakes, iron oxides, natural colors | 0 - 40 | 10 - 30 |
| | Glidant | Talc, lecithin | 0 - 15 | 5 - 15 |

| | | | | |
|---|---|---|---|---|
| * of levels that it may be used (% w/w of total coating material, core tablet weight, or total tablet weight) | | | | |

**Table 12**

| **Proposed Composition of an Immediate Release Tablet Formulation with an Amine Compound in the Core of the Tablet** | | | | |
|---|---|---|---|---|
| **Location in tablet** | **Functional class** | **Examples of components** | **Range*** | **Preferred Range*** |
| Core | Active pharmaceutical ingredient | Saxagliptin, sitagliptin, metformin | 1 - 98 | 5 - 65 |
| | Filler | Lactose monohydrate, microcrystalline cellulose | 5 - 95 | 20 - 80 |
| | Binder | Hydroxypropyl cellulose (HPC), polyvinyl pyrrolidone (PVP), starch, hydroxypropyl methylcellulose (HPMC) | 1 - 20 | 2 - 12 |
| | Disintegrant | Croscarmellose sodium, crospovidone, starch, sodium starch glycollate | 1 - 20 | 2 - 12 |
| | Amine Compound | Glycine, lysine, histidine, arginine, ethanolamine, diethanolamine or triethanolamine or a combination thereof | so as to result in a molar ratio of amine to active ingredient in the range of 1:1 to 20:1 | so as to result in a molar ratio of amine to active ingredient in the range of 1:1 to 10:1 |
| | Glidant | Talc, colloidal silicon dioxide | 0.1 - 10 | 0.5 - 7.5 |
| | Lubricant | Magnesium stearate, stearic acid, | 0.1 - 10 | 0.5 - 2.5 |
| Coating | Polymer | Hydroxypropyl methylcellulose (HPMC), polyvinyl alcohol (PVA), ethyl cellulose (EC), methyl cellulose (MC), hydroxypropyl cellulose (HPC), acrylate polymers (e.g., Eudragits) | 25 - 55 | 35 - 45 |
| | Plasticizer | Polyethylene glycol (PEG), glycerin, castor oil, propyl gallate (PG), surfactants | 1 - 50 | 20 - 30 |
| | Opacifier | Titanium dioxide, pigment, aluminum lakes, iron oxides, natural colors | 0 - 40 | 10 - 30 |
| | Glidant | Talc, lecithin | 0 - 15 | 5 - 15 |

| | | | | |
|---|---|---|---|---|
| * of levels that it may be used (% w/w of total coating material, core tablet weight, or total tablet weight) | | | | |

**Table 13**

| **Proposed Composition of an Immediate Release Tablet Formulation with an Amine Compound in the Coating of the Tablet** | | | | |
|---|---|---|---|---|
| **Location in tablet** | **Functional class** | **Examples of components** | **Range*** | **Preferred Range*** |
| Core | Active pharmaceutical ingredient | Saxagliptin, sitagliptin, metformin | 1 - 98 | 5 - 65 |
| | Filler | Lactose monohydrate, microcrystalline cellulose | 5 - 95 | 20 - 80 |
| | Binder | Hydroxypropyl cellulose (HPC), polyvinyl pyrrolidone (PVP), starch, hydroxypropyl methylcellulose (HPMC) | 1 - 20 | 2 - 12 |
| | Disintegrant | Croscarmellose sodium, crospovidone, starch, sodium starch glycollate | 1 - 20 | 2 - 12 |
| | Glidant | Talc, colloidal silicon dioxide | 0.1 - 10 | 0.5 - 7.5 |
| | Lubricant | Magnesium stearate, stearic acid, | 0.1 - 10 | 0.5 - 2.5 |
| Coating | Polymer | Hydroxypropyl methylcellulose (HPMC), polyvinyl alcohol (PVA), ethyl cellulose (EC), methyl cellulose (MC), hydroxypropyl cellulose (HPC), acrylate polymers (e.g., Eudragits) | 25 - 55 | 35 - 45 |
| | Plasticizer | Polyethylene glycol (PEG), glycerin, castor oil, propyl gallate (PG), surfactants | 1 - 50 | 20 - 30 |
| | Opacifier | Titanium dioxide, pigment, aluminum lakes, iron oxides, natural colors | 0 - 40 | 10 - 30 |
| | Amine Compound | Glycine, lysine, histidine, arginine, ethanolamine, diethanolamine or triethanolamine or a combination thereof | so as to result in a molar ratio of amine to active ingredient in the range of 1:1 to 20:1 | so as to result in a molar ratio of amine to active ingredient in the range of 1:1 to 10:1 |
| | Glidant | Talc, lecithin | 0 - 15 | 5 - 15 |

| | | | | |
|---|---|---|---|---|
| * of levels that it may be used (% w/w of total coating material, core tablet weight, or total tablet weight) | | | | |

**Table 14**

| **Proposed Composition of an Immediate Release Tablet Formulation with Water Soluble Antioxidant(s) in both the Core and the Coating of the Tablet** | | | | |
|---|---|---|---|---|
| **Location in tablet** | **Functional class** | **Examples of components** | **Range*** | **Preferred Range*** |
| Core | Active pharmaceutical ingredient | Saxagliptin, sitagliptin, metformin | 1 - 98 | 5 - 65 |
| | Filler | Lactose monohydrate, microcrystalline cellulose | 5 - 95 | 20 - 80 |
| | Binder | Hydroxypropyl cellulose (HPC), polyvinyl pyrrolidone (PVP), starch, hydroxypropyl methylcellulose (HPMC) | 1 - 20 | 2 - 12 |
| | Disintegrant | Croscarmellose sodium, crospovidone, starch, sodium starch glycollate | 1 - 20 | 2 - 12 |
| | Amine Compound | Glycine, lysine, histidine, arginine, ethanolamine, diethanolamine or triethanolamine or a combination thereof | so as to result in a molar ratio of amine to active ingredient in the range of 1:1 to 20:1 | so as to result in a molar ratio of amine to active ingredient in the range of 1:1 to 10:1 |
| | Glidant | Talc, colloidal silicon dioxide | 0.1 - 10 | 0.5 - 7.5 |
| | Lubricant | Magnesium stearate, stearic acid, | 0.1 - 10 | 0.5 - 2.5 |
| Coating | Polymer | Hydroxypropyl methylcellulose (HPMC), polyvinyl alcohol (PVA), ethyl cellulose (EC), methyl cellulose (MC), hydroxypropyl cellulose (HPC), acrylate polymers (e.g., Eudragits) | 25 - 55 | 35 - 45 |
| | Plasticizer | Polyethylene glycol (PEG), glycerin, castor oil, propyl gallate (PG), surfactants | 1 - 50 | 20 - 30 |
| | Opacifier | Titanium dioxide, pigment, aluminum lakes, iron oxides, natural colors | 0 - 40 | 10 - 30 |
| | Amine Compound | Glycine, lysine, histidine, arginine, ethanolamine, diethanolamine or triethanolamine or a combination thereof | so as to result in a molar ratio of amine to active ingredient in the range of 1:1 to 20:1 | so as to result in a molar ratio of amine to active ingredient in the range of 1:1 to 10:1 |
| | Glidant | Talc, lecithin | 0 - 15 | 5 - 15 |

| | | | | |
|---|---|---|---|---|
| * of levels that it may be used (% w/w of total coating material, core tablet weight, or total tablet weight) | | | | |

### Example 11

### Saxagliptin Tablet

A saxagliptin tablet is prepared by coating a placebo core tablet with polyvinyl alcohol (PVA)-based Opadry® coating material in a three layer coating process to embed saxagliptin in the coating material. A formulation composition of a saxagliptin tablet according to this invention is listed in Table 15. This composition reflects a saxagliptin dose of 2.5 mgs. Saxagliptin tablets of different doses can be prepared by modifying the API loading and composition of the excipients and total tablet weight.

**Table 15**

| **Composition of Saxagliptin Tablet (2.5 mg)** | | |
|---|---|---|
| **Material** | **Function** | **Quantity in Mg/tablet** |
| **Core tablet composition:** | | |
| Lactose monohydrate | Filler | 99 |
| Microcrystalline cellulose | Filler | 90 |
| Croscarmellose sodium | Disintegrant | 10 |
| Magnesium Stearate | Lubricant | 1 |

| **First Layer Composition:** | | |
|---|---|---|
| Opadry® II White | Coating Material | 21 |

| **Second Layer Composition:** | | |
|---|---|---|
| Saxagliptin | API | 2.5 |
| Opadry® II White | Coating Material | 20 |
| Glycine | Amine Compound | 5.9 |

| **Third Layer Composition:** | | |
|---|---|---|
| Opadry® II color | Coating Material | 17 |
| | | |
| Hydrochloric Acid Solution, 1N | For pH adjustment | q.s. |
| Purified Water | Vehicle for coating suspension | q.s. |
| Sodium Hydroxide Solution, IN | For pH adjustment | q.s. |
| Opacode | Printing Ink | 0.03 |

### Example 12

### Saxagliptin/Metformin Immediate Release Fixed Dose Tablet

A saxagliptin/metformin hydrochloride immediate release (Saxa/Met IR) tablet is prepared by coating a Met IR core tablet with PVA-based Opadry® coating material in a three layer coating process to embed saxagliptin in the coating material. A formulation composition of the Saxa/Met IR tablet is listed in Table 16. This composition reflects saxagliptin dose of 2.5 mg and metformin hydrochloride dose of 500 mg. Other fixed dose combination products can be prepared by modifying the API loading and composition of the excipients in the core or the coating of the tablet and total tablet weight.

**Table 16**

| **Composition of Saxa/Met IR Tablet** | | |
|---|---|---|
| **Material** | **Function** | **Quantity in Mg/tablet** |
| **Core tablet composition:** | | |
| Metformin Hydrochloride | API | 500 |
| Povidone | Binder | 20 |
| Purified Water ca. | Wetting agent | 5 |
| Magnesium Stearate | Lubricant | 1.75 |

| **First Layer Composition:** | | |
|---|---|---|
| Opadry II White | Coating Material | 21 |

| **Second Layer Composition:** | | |
|---|---|---|
| Saxagliptin | API | 2.5 |
| Opadry® II White | Coating Material | 20 |
| Glycine | Amine Compound | 5.9 |

| **Third Layer Composition:** | | |
|---|---|---|
| Opadry II color | Coating Material | 17 |
| | | |
| Hydrochloric Acid Solution, 1N | For pH adjustment | q.s. |
| Purified Water | Vehicle for coating suspension | q.s. |
| Sodium Hydroxide Solution, IN | For pH adjustment | q.s. |
| Opacode | Printing Ink | 0.03 |

### Example 13

### Saxagliptin/Metformin Extended Release Fixed Dose Tablet

A saxagliptin/metformin hydrochloride extended release (Saxa/Met XR) tablet is prepared by coating a Met XR core tablet with PVA-based Opadry® coating material in a three layer coating process to embed saxagliptin in the coating material. A formulation composition of the Saxa/Met XR tablet is listed in Table 17. This composition reflects saxagliptin dose of 2.5 mg and metformin hydrochloride dose of 500 mg. Other fixed dose combination products can be prepared by modifying the API loading and composition of the excipients in the core or the coating of the tablet and total tablet weight.

**Table 17**

| **Composition of Saxa/Met XR Tablet** | | |
|---|---|---|
| **Material** | **Function** | **Quantity in Mg/tablet** |
| **Core tablet composition:** | | |
| Metformin Hydrochloride | API | 500 |
| Sodium carboxymethylcellulose | Disintegrant | 50 |
| Hydroxypropyl methylcellulose | Release modifier | 370 |
| Microcrystalline cellulose | Filler | 100 |
| Purified Water ca. | Wetting agent | 5 |
| Magnesium Stearate | Lubricant | 3.5 |

| **First Layer Composition:** | | |
|---|---|---|
| Opadry II White | Coating Material | 21 |

| **Second Layer Composition:** | | |
|---|---|---|
| Saxagliptin | API | 2.5 |
| Opadry® II White | Coating Material | 20 |
| Glycine | Amine Compound | 5.9 |

| **Third Layer Composition:** | | |
|---|---|---|
| Opadry II color | Coating Material | 17 |
| | | |
| Hydrochloric Acid Solution, 1N | For pH adjustment | q.s. |
| Purified Water | Vehicle for coating suspension | q.s. |
| Sodium Hydroxide Solution, IN | For pH adjustment | q.s. |
| Opacode | Printing Ink | 0.03 |

### Reference Example 14

### Liquid and Semi-solid Formulations

The formulations of the present invention are also applicable to liquid formulations, which may be intended for oral, external, or parenteral use. These formulations can include solutions, suspensions, emulsions, ointments, gels, suppositories, self-emulsifying systems, self-microemulsifying systems, and other semi-solid dosage forms. The liquid formulations of this invention can be used for drugs that react with formic acid or a formylating species, such as formic acid esters or anhydrides. Amine compounds may be added to formulations that contain residual levels of formic acid or a different formylating species (e.g., in the range of 5 ppm or higher). The formulations may contain a formylating species initially and/or they may be formed on storage at high temperature (such as 40 °C to 60 °C) and/or humidity (such as 75% RH to 95% RH) conditions over a period of 2 days to 3 months.

## Claims

1. A coated tablet comprising:
(a) a tablet core wherein the tablet core comprises
(i) optionally at least one antidiabetic agent or a pharmaceutically acceptable salt thereof selected from the group consisting of glyburide, metformin, rosiglitazone, pioglitazone, sitagliptin, vildagliptin, dapagliflozin, and dapagliflozin (S) propylene glycol hydrate;
(b) an active coating layer surrounding the tablet core wherein the coating layer comprises, in admixture,
(i) a coating material comprising poly(ethylene glycol) and poly(vinyl alcohol);
(ii) an amine compound that is glycine, histidine, arginine, ethanolamine, diethanolamine or triethanolamine, or a combination thereof; and
(iii) saxagliptin or a pharmaceutically acceptable salt thereof.

2. The coated tablet according to claim 1, wherein the amine compound is histidine or arginine.

3. The coated tablet according to claim 1, wherein the amine compound is glycine.

4. The coated tablet according to any of claims 1-3, wherein the amine compound is substantially provided as an ammonium salt.

5. The coated tablet according to any of claims 1-4, wherein the amine compound is provided at a concentration in the range of 0.01 wt% to 10 wt% of the coating layer.

6. The coated tablet according to claim 5, wherein the amine compound is provided at a concentration in the range of 0.5 wt% to 5 wt% of the coating layer.

7. The coated tablet according to any of claims 1-4, wherein the molar ratio of the amine compound to saxagliptin is in the range of 1:1 to 100:1.

8. The coated tablet according to any of claims 1-4, wherein the molar ratio of the amine compound to saxagliptin is in the range of 1:1 to 10:1.

9. The coated tablet according to any of claims 1-8, wherein the coating material comprises polyvinyl alcohol, titanium dioxide, polyethylene glycol, and talc.

10. The coated tablet according to any of claims 1-9, wherein saxagliptin is substantially provided as an ammonium salt.

11. The coated tablet according any of claims 1-10, wherein the coating layer further comprises a water soluble antioxidant.

12. The coated tablet according to claim 11 wherein the water soluble antioxidant is ascorbic acid or propyl gallate.

13. The coated tablet according to any of claims 1-12, wherein the tablet core comprises one or more fillers, a disintegrant, and a lubricant.

14. The coated tablet according to any of claims 1-13 wherein the tablet core comprises a binder, a wetting agent, a lubricant, and at least one of the antidiabetics or the pharmaceutically acceptable salts thereof.

15. The coated tablet according to any of claims 1-14 wherein the tablet core comprises a disintegrant, a release modifier, a filler, a wetting agent, a lubricant, and at least one of the antidiabetics or the pharmaceutically acceptable salts thereof.

16. The coated tablet according to claim 14 or claim 15 wherein the antidiabetic is metformin, dapagliflozin, dapagliflozin (S) propylene glycol hydrate, or a combination thereof.

17. The coated tablet according to any of claims 1-16, wherein the active coating layer is at least 25% total of poly(ethylene glycol) and poly(vinyl alcohol).

18. The coated tablet according to any of claims 1-16, wherein the active coating layer is at least 50% total of poly(ethylene glycol) and poly(vinyl alcohol).

19. The coated tablet according to any of claims 1-18, wherein the coated tablet comprises:
(a) the tablet core;
(b) a first layer that coats the tablet core, and is coated by the coating layer, wherein the first layer comprises
(i) a coating material; and
(ii) optionally an amine compound that is glycine, histidine, arginine, ethanolamine, diethanolamine or triethanolamine, or a combination thereof;
(c) the active coating layer provided as a second layer that coats the first layer; and
(d) a third layer that coats the second layer wherein the third layer comprises
(i) a coating material; and
(ii) optionally an amine compound that is glycine, histidine, arginine, ethanolamine, diethanolamine or triethanolamine, or a combination thereof.

## Patentansprüche

1. Beschichtete Tablette, die Folgendes umfasst:
(a) einen Tablettenkern, wobei der Tablettenkern Folgendes umfasst:
(i) gegebenenfalls zumindest ein Antidiabetesmittel oder ein pharmazeutisch annehmbares Salz davon, ausgewählt aus der aus Glyburid, Metformin, Rosiglitazon, Pioglitazon, Sitagliptin, Vildagliptin, Dapagliflozin und Dapagliflozin-(S)-propylenglykolhydrat bestehenden Gruppe; und
(b) eine aktive Beschichtungsschicht, die den Tablettenkern umgibt, wobei die Beschichtungsschicht Folgendes als Gemisch umfasst:
(i) ein Beschichtungsmaterial, das Polyethylenglykol und Polyvinylalkohol umfasst;
(ii) eine Aminverbindung, die Glycin, Histidin, Arginin, Ethanolamin, Diethanolamin oder Triethanolamin oder eine Kombination davon ist; und
(iii) Saxagliptin oder ein pharmazeutisch annehmbares Salz davon.

2. Beschichtete Tablette nach Anspruch 1, wobei die Aminverbindung Histidin oder Arginin ist.

3. Beschichtete Tablette nach Anspruch 1, wobei die Aminverbindung Glycin ist.

4. Beschichtete Tablette nach einem der Ansprüche 1 bis 3, wobei die Aminverbindung im Wesentlichen als Ammoniumsalz bereitgestellt ist.

5. Beschichtete Tablette nach einem der Ansprüche 1 bis 4, wobei die Aminverbindung in einer Konzentration im Bereich von 0,01 Gew.-% bis 10 Gew.-% der Beschichtungsschicht bereitgestellt ist.

6. Beschichtete Tablette nach Anspruch 5, wobei die Aminverbindung in einer Konzentration im Bereich von 0,5 Gew.-% bis 5 Gew.-% der Beschichtungsschicht bereitgestellt ist.

7. Beschichtete Tablette nach einem der Ansprüche 1 bis 4, wobei das Molverhältnis zwischen der Aminverbindung und Saxagliptin im Bereich von 1:1 bis 100:1 liegt.

8. Beschichtete Tablette nach einem der Ansprüche 1 bis 4, wobei das Molverhältnis zwischen der Aminverbindung und Saxagliptin im Bereich von 1:1 bis 10:1 liegt.

9. Beschichtete Tablette nach einem der Ansprüche 1 bis 8, wobei das Beschichtungsmaterial Polyvinylalkohol, Titandioxid, Polyethylenglykol und Talk umfasst.

10. Beschichtete Tablette nach einem der Ansprüche 1 bis 9, wobei Saxagliptin im Wesentlichen als Ammoniumsalz bereitgestellt ist.

11. Beschichtete Tablette nach einem der Ansprüche 1 bis 10, wobei die Beschichtungsschicht weiters ein wasserlösliches Antioxidans umfasst.

12. Beschichtete Tablette nach Anspruch 11, wobei das wasserlösliche Antioxidans Ascorbinsäure oder Propylgallat ist.

13. Beschichtete Tablette nach einem der Ansprüche 1 bis 12, wobei der Tablettenkern einen oder mehrere Füllstoffe, ein Sprengmittel und ein Gleitmittel umfasst.

14. Beschichtete Tablette nach einem der Ansprüche 1 bis 13, wobei der Tablettenkern ein Bindemittel, ein Benetzungsmittel, ein Gleitmittel und zumindest eines der Antidiabetesmittel oder der pharmazeutisch annehmbaren Salze davon umfasst.

15. Beschichtete Tablette nach einem der Ansprüche 1 bis 14, wobei der Tablettenkern ein Sprengmittel, einen Freisetzungsmodifikator, einen Füllstoff, ein Benetzungsmittel, ein Gleitmittel und zumindest eines der Antidiabetesmittel oder der pharmazeutisch annehmbaren Salze davon umfasst.

16. Beschichtete Tablette nach Anspruch 14 oder Anspruch 15, wobei das Antidiabetesmittel Metformin, Dapagliflozin-(S)-propylenglykolhydrat oder eine Kombination davon ist.

17. Beschichtete Tablette nach einem der Ansprüche 1 bis 16, wobei die aktive Beschichtungsschicht insgesamt zumindest zu 25 % aus Poly(ethylenglykol) und Poly(vinylalkohol) besteht.

18. Beschichtete Tablette nach einem der Ansprüche 1 bis 16, wobei die aktive Beschichtungsschicht insgesamt zumindest zu 50 % aus Poly(ethylenglykol) und Poly(vinylalkohol) besteht.

19. Beschichtete Tablette nach einem der Ansprüche 1 bis 18, wobei die beschichtete Tablette Folgendes umfasst:
(a) den Tablettenkern;
(b) eine erste Schicht, die den Tablettenkern einhüllt und mit der Beschichtungsschicht beschichtet ist, wobei die erste Schicht Folgendes umfasst:
(i) ein Beschichtungsmaterial; und
(ii) gegebenenfalls eine Aminverbindung, die Glycin, Histidin, Arginin, Ethanolamin, Diethanolamin oder Triethanolamin oder eine Kombination davon ist;
(c) die aktive Beschichtungsschicht, die als zweite Schicht bereitgestellt ist, die die erste Schicht einhüllt; und
(d) eine dritte Schicht, die die zweite Schicht einhüllt, wobei die dritte Schicht Folgendes umfasst:
(i) ein Beschichtungsmaterial; und
(ii) gegebenenfalls eine Aminverbindung, die Glycin, Histidin, Arginin, Ethanolamin, Diethanolamin oder Triethanolamin oder eine Kombination davon ist.

## Revendications

1. Comprimé enrobé comprenant :
(a) un noyau de comprimé dans lequel le noyau de comprimé comprend
(i) facultativement au moins un agent antidiabétique ou un sel pharmaceutiquement acceptable de celui-ci choisi parmi le groupe constitué de glyburide, metformine, rosiglitazone, pioglitazone, sitagliptine, vildagliptine, dapagliflozine, et propylène glycol hydraté de dapagliflozine (S) ;
(b) une couche d'enrobage active entourant le noyau de comprimé, la couche d'enrobage comprenant, en mélange,
(i) une matière d'enrobage comprenant du poly(éthylène glycol) et du poly(alcool vinylique) ;
(ii) un composé amine qui est de la glycine, de l'histidine, de l'arginine, de l'éthanolamine, de la diéthanolamine ou de la triéthanolamine, ou une combinaison de celles-ci ; et
(iii) de la saxagliptine ou un sel pharmaceutiquement acceptable de celle-ci.

2. Comprimé enrobé selon la revendication 1, dans lequel le composé amine est de l'histidine ou de l'arginine.

3. Comprimé enrobé selon la revendication 1, dans lequel le composé amine est de la glycine.

4. Comprimé enrobé selon l'une quelconque des revendications 1 à 3, dans lequel le composé amine est essentiellement fourni sous forme d'un sel d'ammonium.

5. Comprimé enrobé selon l'une quelconque des revendications 1 à 4, dans lequel le composé amine est fourni à une concentration dans la plage de 0,01 % en poids à 10 % en poids de la couche d'enrobage.

6. Comprimé enrobé selon la revendication 5, dans lequel le composé amine est fourni à une concentration dans la plage de 0,5 % en poids à 5 % en poids de la couche d'enrobage.

7. Comprimé enrobé selon l'une quelconque des revendications 1 à 4, dans lequel le rapport molaire entre le composé amine et la saxagliptine est dans la plage de 1:1 à 100:1.

8. Comprimé enrobé selon l'une quelconque des revendications 1 à 4, dans lequel le rapport molaire entre le composé amine et la saxagliptine est dans la plage de 1:1 à 10:1.

9. Comprimé enrobé selon l'une quelconque des revendications 1 à 8, dans lequel la matière d'enrobage comprend de l'alcool polyvinylique, du dioxyde de titane, du polyéthylène glycol, et du talc.

10. Comprimé enrobé selon l'une quelconque des revendications 1 à 9, dans lequel la saxagliptine est essentiellement fournie sous forme d'un sel d'ammonium.

11. Comprimé enrobé selon l'une quelconque des revendications 1 à 10, dans lequel la couche d'enrobage comprend en outre un antioxydant soluble dans l'eau.

12. Comprimé enrobé selon la revendication 11, dans lequel l'antioxydant soluble dans l'eau est de l'acide ascorbique ou du gallate de propyle.

13. Comprimé enrobé selon l'une quelconque des revendications 1 à 12, dans lequel le noyau de comprimé comprend une ou plusieurs charges, un désintégrant, et un lubrifiant.

14. Comprimé enrobé selon l'une quelconque des revendications 1 à 13, dans lequel le noyau de comprimé comprend un liant, un agent mouillant, un lubrifiant, et au moins l'un parmi les antidiabétiques ou leurs sels pharmaceutiquement acceptables.

15. Comprimé enrobé selon l'une quelconque des revendications 1 à 14, dans lequel le noyau de comprimé comprend un désintégrant, un modificateur de libération, une charge, un agent mouillant, un lubrifiant, et au moins l'un parmi les antidiabétiques ou leurs sels pharmaceutiquement acceptables.

16. Comprimé enrobé selon la revendication 14 ou la revendication 15, dans lequel l'antidiabétique est de la metformine, de la dapagliflozine, du propylène glycol hydraté de dapagliflozine (S), ou une combinaison de ceux-ci.

17. Comprimé enrobé selon l'une quelconque des revendications 1 à 16, dans lequel la couche d'enrobage active est constituée d'au moins 25 % au total de poly(éthylène glycol) et de poly(alcool vinylique).

18. Comprimé enrobé selon l'une quelconque des revendications 1 à 16, dans lequel la couche d'enrobage active est constituée d'au moins 50 % au total de poly(éthylène glycol) et de poly(alcool vinylique).

19. Comprimé enrobé selon l'une quelconque des revendications 1 à 18, dans lequel le comprimé enrobé comprend :
(a) le noyau de comprimé ;
(b) une première couche qui enrobe le noyau de comprimé, et est enrobée par la couche d'enrobage, la première couche comprenant
(i) une matière d'enrobage ; et
(ii) facultativement un composé amine qui est de la glycine, de l'histidine, de l'arginine, de l'éthanolamine, de la diéthanolamine ou de la triéthanolamine, ou une combinaison de celles-ci ;
(c) la couche d'enrobage active fournie en tant que deuxième couche qui enrobe la première couche ; et
(d) une troisième couche qui enrobe la deuxième couche, la troisième couche comprenant
(i) une matière d'enrobage ; et
(ii) facultativement un composé amine qui est de la glycine, de l'histidine, de l'arginine, de l'éthanolamine, de la diéthanolamine ou de la triéthanolamine, ou une combinaison de celles-ci.
